(19) Europäisches Patentamt
European Patent Office
Office européen des brevets

(11) **EP 1 917 901 B1**

(12) **EUROPEAN PATENT SPECIFICATION**

(45) Date of publication and mention of the grant of the patent:
**17.04.2019 Bulletin 2019/16**

(21) Application number: **06781486.3**

(22) Date of filing: **24.07.2006**

(51) Int Cl.:
*A61B 1/00* *(2006.01)*     *G02B 23/24* *(2006.01)*
*A61B 5/06* *(2006.01)*

(86) International application number:
**PCT/JP2006/314574**

(87) International publication number:
**WO 2007/023631 (01.03.2007 Gazette 2007/09)**

(54) **ENDOSCOPE INSERTION SHAPE ANALYSIS APPARATUS AND ENDOSCOPE INSERTION SHAPE ANALYSIS SYSTEM**

VORRICHTUNG ZUR ANALYSE EINER ENDOSKOP-EINFÜHRUNGSFORM UND SYSTEM ZUR ANALYSE EINER ENDOSKOP-EINFÜHRUNGSFORM

DISPOSITIF D'ANALYSE DE FORME D'INSERTION D'ENDOSCOPE ET SYSTÈME D'ANALYSE DE FORME D'INSERTION D'ENDOSCOPE

(84) Designated Contracting States:
**DE FR GB**

(30) Priority: **25.08.2005 JP 2005244686**
**16.11.2005 JP 2005332009**

(43) Date of publication of application:
**07.05.2008 Bulletin 2008/19**

(73) Proprietor: **Olympus Corporation**
**Tokyo 192-8507 (JP)**

(72) Inventor: **TANAKA, Hideki**
**Hachioji-shi,**
**Tokyo 192-8507 (JP)**

(74) Representative: **von Hellfeld, Axel et al**
**Wuesthoff & Wuesthoff**
**Patentanwälte PartG mbB**
**Schweigerstrasse 2**
**81541 München (DE)**

(56) References cited:
**EP-A1- 1 504 712     JP-A- 2004 147 778**
**JP-A- 2004 358 095     JP-A- 2005 328 999**

**Description**

Technical Field

The present invention relates to an endoscope insertion shape analysis apparatus and an endoscope insertion shape analysis system for analyzing a situation in which insertion of an endoscope insertion portion is inhibited, for example, at the time of insertion.

Background Art

A lumen in a body cavity as a subject at the time of observation by inserting an endoscope is curved, for example like large intestine, small intestine, or the like. Therefore, detecting to which position in a lumen an endoscope insertion portion is inserted and what shape the endoscope insertion portion takes leads to improvement in operability of observation treatment by the endoscope.

Therefore, there has been conventionally proposed an endoscope shape detecting apparatus for detecting an insertion shape of an endoscope by using a source coil and the like, as an apparatus to detect a flexion state and the like at the time of insertion of the endoscope.

During observation of a subject by an endoscope, an operator tends to concentrate his or her awareness mainly on an endoscope image generated by picking up an image of a region to be observed in a lumen. Accordingly, the operator does not often concentrate his or her awareness on an image of an insertion shape generated and displayed by an endoscope shape detecting apparatus. The operator does not pay attention to the image of insertion shape until hindrance occurs in a course of insertion of an endoscope insertion portion. This will be a factor to disturb a course of endoscope observation and cause a subject to feel a sense of discomfort.

In addition, generally in an endoscope observation, endoscope images are recorded and used later for confirmation of a region to be observed and a training for acquiring endoscope operation technique.

To this end, in Japanese Unexamined Patent Application Publication No. 2004-147778, there is proposed an endoscope image processing apparatus capable of saving both endoscope insertion shape data and endoscope image data and freely comparing the images by synchronously reproducing both of the images.

The apparatus proposed in the Japanese Unexamined Patent Application Publication No. 2004-147778 detects a shape of endoscope insertion portion when the endoscope is inserted into a large intestine, for example, by using an endoscope insertion shape observation apparatus and analyzes the shape by an image processing apparatus to provide analysis result on the shape of endoscope insertion portion.

The analysis result relates to the shape of insertion portion and endoscope operation by an operator and is obtained by determining whether or not the shape of the endoscope insertion portion is a predetermined shape and whether or not the shape shows a predetermined change.

In addition, it is more convenient to grasp, as insertion aid information, information on whether or not the insertion portion appropriately responds to the insertion operation when the insertion operation is actually performed at the hand side of the insertion portion in addition to the information on insertion shape and the like.

For example, in a conventional example disclosed in Japanese Unexamined Patent Application Publication No. 2004-358095, the apparatus regularly analyzes whether or not the insertion portion has become a loop shape, and displays the shape information when the insertion portion has become a loop shape. Furthermore, the apparatus also analyzes that the distal end side of the insertion portion is in a stop state.

Document EP 1 504 712 A1 discloses an endoscope information processor and processing method comprising a shape analysing unit that analyses an insertional shape acquired by detecting the shape of an insertion unit of an endoscope which is inserted into a body cavity, and an information providing unit that provides information on the situation of handling the endoscope according to the result of the analysis performed by the shape analysing unit.

Disclosure of Invention

Means for Solving the Problem

However, there are problems as described below in the analysis result and the method for drawing the analysis result provided by the apparatus disclosed in Japanese Unexamined Patent Application Publication No. 2004-147778. For example, one of the problems is that an erroneous determination occurs due to the simple determination of whether or not to satisfy a predetermined condition (for example, the apparatus cannot grasp the whole shape of insertion portion as a result of picking up local characteristics (an angle and the like)). Another problem is that the apparatus is vulnerable to disturbance since the apparatus determines the shape by an instantaneous shape at one clock time.

In addition, in the conventional example disclosed in above-described Japanese Unexamined Application Publication No. 2004-358095 , the operator can grasp the shape information if he or she performs observation at a timing when the shape of insertion portion is analyzed as a loop shape and the shape is displayed. However, there is a drawback in the conventional apparatus such that the operator overlooks the information if he or she performs observation, missing the timing around when the shape of the insertion portion has been analyzed as a loop-shape, because the display of the shape is regularly updated.

The present invention has been achieved in view of the above circumstances and an object of the present invention is to provide an endoscope insertion shape analysis apparatus capable of performing stable shape analysis and analyzing endoscope insertion state with a whole shape of an insertion portion.

Another object of the present invention is to provide an endoscope insertion shape analysis apparatus and an endoscope insertion shape analysis system capable of more securely confirming insertion aid information corresponding to a predetermined response action state of an insertion portion with respect to insertion operation and the like.

Brief Description of the Drawings

Some or all of the above problems are overcome by an endoscope insertion shape analysis apparatus having the features of claim 1.

FIG. 1 is a block diagram showing a whole configuration of an electronic endoscope system according to a first embodiment of the present invention.

FIG. 2 is a relationship diagram showing an insertion portion of an endoscope of FIG. 1 in which source coils are incorporated and coordinates of the source coils.

FIG 3 is a data structure diagram showing a structure of insertion shape data generated by the endoscope insertion shape observation apparatus.

FIG. 4 is a diagram showing a configuration of analysis windows displayed on a display of an image processing apparatus of FIG. 1.

FIG. 5 is a flowchart diagram related to processings of inspection information and endoscope image, and insertion shape data in the image processing apparatus of FIG. 1.

FIG. 6 is a detailed flowchart diagram of shape analysis processing in FIG. 5.

FIG. 7 is a diagram describing the processings in FIG. 6.

FIG. 8 is a diagram showing a configuration of a dictionary file stored in a storage device in the image processing apparatus of FIG. 1.

FIG. 9 is a first diagram showing a shape classified using the dictionary file of FIG. 8.

FIG. 10 is a second diagram showing a shape classified using the dictionary file of FIG. 8.

FIG. 11 is a third diagram showing a shape classified using the dictionary file of FIG. 8.

FIG. 12 is a fourth diagram showing a shape classified using the dictionary file of FIG. 8.

FIG. 13 is a fifth diagram showing a shape classified using the dictionary file of FIG. 8.

FIG. 14 is a diagram showing a ring buffer established in a memory of an image processing apparatus according to a second embodiment of the present invention.

FIG. 15 is a flowchart describing a flow of processings of the image processing apparatus using the ring buffer of FIG. 14.

FIG. 16 is a diagram describing the processings of FIG. 15.

FIG. 17 is a diagram showing a dictionary file available in the processings of FIG. 15 which is stored in a storage device of the image processing apparatus.

FIG. 18 is a diagram showing a code correction dictionary available in the processings of FIG. 15 which is stored in the storage device of the image processing apparatus.

FIG. 19 is a block diagram showing a whole configuration of an electronic endoscope system according to a third embodiment of the present invention.

FIG. 20 is a block diagram showing a configuration of a running program of a personal computer in the image processing apparatus of FIG. 19.

FIG. 21 is a diagram showing a registration window developed by an output destination registration block of the running program of FIG. 20.

FIG. 22 is a first flowchart showing a flow of the processings by the running program of FIG 20.

FIG. 23 is a second flowchart showing a flow of the proecessings by the running program of FIG. 20.

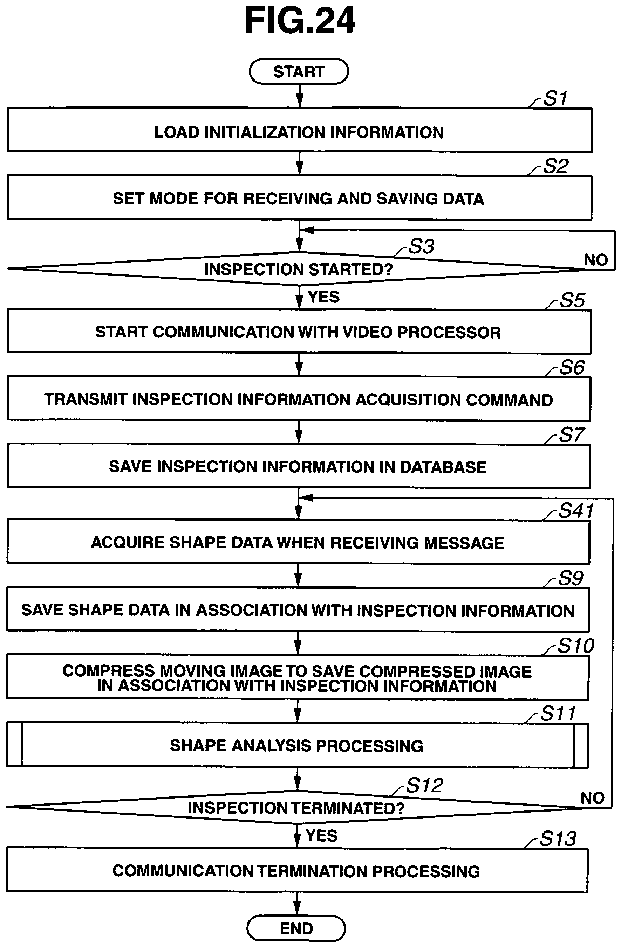

FIG. 24 is a third flowchart showing a flow of the processings by the running program of FIG. 20.

FIG. 25 is a diagram showing a whole configuration of an in-vivo insertion monitoring system provided with a fourth embodiment of the present invention.

FIG. 26 is a diagram showing coordinates of source coils provided in an insertion portion to be detected by an endoscope insertion shape observation apparatus according to the fourth embodiment of the present invention.

FIG. 27 is an explanatory diagram showing insertion shape data generated by the endoscope insertion shape observation apparatus according to the fourth embodiment of the present invention.

FIG. 28 is a diagram showing configurations and the like of function blocks realized by an image processing apparatus according to the fourth embodiment of the present invention.

FIG. 29 is an explanatory diagram showing a data flow, a processing flow, and the like by an analysis processing block and the like shown in FIG. 28.

FIG. 30 is an explanatory diagram showing action for controlling display maintenance of insertion aid information based on difference values between updating timing of the insertion aid information according to the fourth embodiment of the present invention and current time.

FIG. 31 is a diagram showing a modification example related to display characteristics of the insertion aid information according to the fourth embodiment of the present invention.

FIG. 32 is a diagram showing configurations and the like of function blocks realized by an image processing apparatus according to a fifth embodiment of the present invention.
FIG. 33 is an explanatory diagram showing a data flow, a processing flow, and the like by an analysis processing block and the like shown in FIG. 32.
FIG. 34 is a specific example of a programming of processing content of processing script according to the fifth embodiment of the present invention.

Best Mode for Carrying Out the Invention

Hereinafter, embodiments of the present invention are described with reference to the drawings.

(First Embodiment)

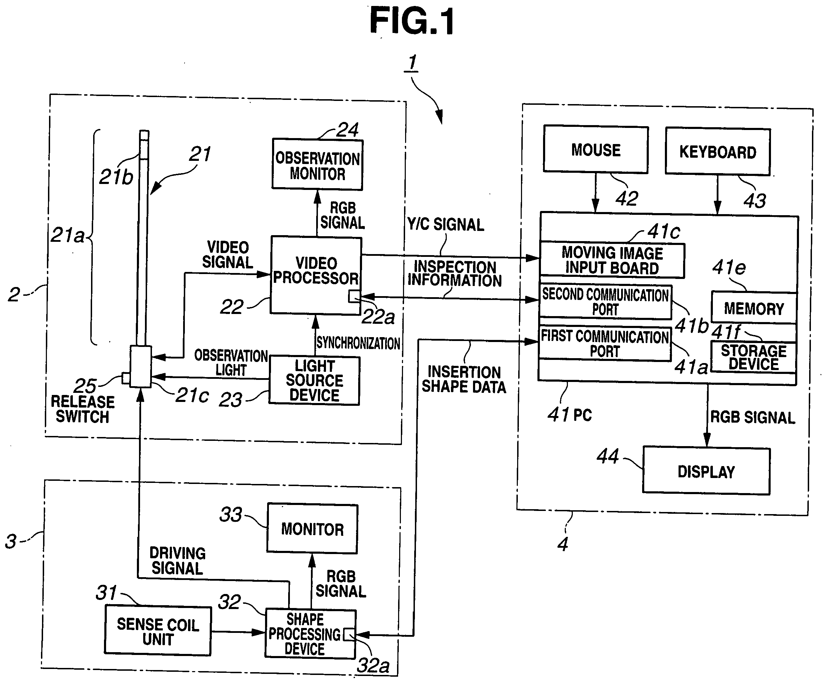

As shown in FIG. 1, an electronic endoscope system 1 as an endoscope insertion shape analysis apparatus includes an endoscope apparatus 2, an endoscope insertion shape observation apparatus 3, and an image processing apparatus 4.
The endoscope apparatus 2 includes an electronic endoscope 21, a video processor 22, a light source device 23, and an observation monitor 24.
The electronic endoscope 21 is configured such that observation light for illuminating a region to be observed in a lumen is irradiated from a distal end portion of an insertion portion 21a by a light guide not shown provided in the insertion portion 21a. The electronic endoscope 21 has an electronic image pickup device not shown (for example, CCD) provided at a distal end of the elongated insertion portion 21a to be inserted into a lumen of a body cavity as a subject. Furthermore, the electronic endoscope 21, by driving and controlling the electronic image pickup device, generates a video signal by picking up an image of a region to be observed in the lumen and outputs the generated video signal.
In addition, the electronic endoscope 21 has a bending portion 21b provided at a distal end part of the insertion portion 21a, and the bending portion 21b can be operated to be bent by an operation portion 21c provided at a proximal end side of the insertion portion 21a.
In addition, the electronic endoscope 21 has a release switch 25 provided to the operation portion 21c. Moreover, the operation portion 21c has, between itself and the video processor 22, a cable for driving and controlling the electronic image pickup device and for transmitting and receiving the video signal generated by image pickup. Also, the operation portion 21c has a light guide cable (not shown) for guiding observation light from the light source device 23 to the light guide, and the like.
In addition, the electronic endoscope 21 includes a plurality of source coils, to be described later, configuring a detection function for detecting an insertion position and a shape of the insertion portion 21a in the lumen. The detection function for detecting the insertion position and shape is configured of a plurality of source coils disposed along an insertion axis of the insertion portion 21a, and a sense coil unit 31 having a plurality of sense coils, which is provided in the endoscope insertion shape observation apparatus 3.
Note that the plurality of source coils are disposed in the insertion portion 21a of an endoscope at predetermined intervals decided depending on a type of the endoscope.
The video processor 22 drives and controls the electronic image pickup device in the electronic endoscope 21. Furthermore, the video processor 22 performs a predetermined signal processing on the video signal of a moving image generated by photoelectric conversion by the electronic image pickup device to generate a Y/C signal composed of luminance signal and chrominance signal, an RGB signal, or the like. The Y/C signal composed of the luminance signal and the chrominance signal or the RGB signal which are generated by the video processor 22 are directly outputted to the observation monitor 24 and the image processing apparatus 4.
In addition, the video processor 22 can give an instruction for outputting a still image of the picked-up image when the release switch 25 of the endoscope 21 is operated.
Note that the video processor 22 includes an input function not shown for inputting inspection information on endoscopy.
The light source device 23 includes a lamp as an illumination light source not shown, a lighting circuit for the lamp not shown, and the like. The light source device 23 supplies to the light guide of the electronic endoscope 21 illumination light projected when the lamp is lighted, and the illumination light is projected from the distal end of the insertion portion 21a onto the region to be observed in the lumen.
The observation monitor 24 displays an endoscope image based on the Y/C signal, the RGB signal, or the like, generated in the video processor 22.
The endoscope insertion shape observation apparatus 3 is a peripheral apparatus of the endoscope apparatus 2 and includes the sense coil unit 31, a shape processing device 32, and a monitor 33. The sense coil unit 31 is a unit for detecting magnetic fields from the plurality of source coils provided in the insertion portion 21a of the electronic endoscope 21. The shape processing device 32 is a device for estimating the shape of the endoscope insertion portion based on the magnetic fields detected by the sense coil unit 31. The monitor 33 is a device for displaying the shape of endoscope insertion portion estimated by the shape processing device 32.
The shape processing device 32 causes the source coils to generate magnetic fields by outputting to the electronic endoscope 21 a driving signal for driving the source coils. The shape processing device 32 calculates positional coordinate data of each of the source coils based on the detection signals from the sense coil unit 31 which has detected the generated magnetic fields, and estimates the shape of the endoscope insertion portion from the calculated positional coordinate data. The shape processing device 32 also generates an insertion portion shape image signal to display the estimated shape of the endoscope insertion portion on the monitor. Moreover, the shape processing device 32 is so configured as to generate insertion shape data such as three-dimensional coordinate information showing the shape of the endoscope insertion portion and shape display attribute to be outputted to the image processing apparatus 4.

As described above, the plurality of source coils, the sense coil unit 31, and the shape processing device 32 configure shape detection means.

Note that, in the endoscope insertion shape observation apparatus 3, the shape display attributes such as rotation angle, magnification/reduction, and the like, of the insertion portion shape image subjected to processing and generated by the shape processing device 32 to be displayed on the monitor 33 can be changed by inputting instruction from an operation panel not shown. Moreover, the insertion shape data generated by the shape processing device 32 can be outputted to the image processing apparatus 4.

The image processing apparatus 4 includes a personal computer (hereinafter called only PC) 41 configuring shape analysis means and pattern classification means, a mouse 42, and a keyboard 43. The mouse 42 and the keyboard 43 are input devices for inputting various instructions to the PC 41. The display 44 is a device for reproducing and displaying various kinds of information data and image information processed by the PC 41. Furthermore, the display 44 as display means displays the endoscope image picked up by the electronic endoscope 21 and the shape of endoscope insertion portion detected by the shape processing device 32 on one screen.

In addition, the PC 41 includes a first communication port 41a, a second communication port 41b, a moving image input board 41c, a memory 41e composed of semiconductor device, for example, and a storage device 41f as information storage means composed of a hard disk, for example. The PC 41 as shape analysis means calculates, that is, analyzes to find a specific position at which the endoscope insertion is blocked, for example, based on the insertion shape data and the like, as described later.

The first communication port 41a is configured to load the insertion shape data outputted from a communication port 32a of the shape processing device 32 in the endoscope insertion shape observation apparatus 3.

The second communication port 41b is configured to load endoscopy information outputted from a communication port 22a of the video processor 22 in the endoscope apparatus 2.

The moving image input board 41c converts the moving image video signal generated by the video processor 22 in the endoscope apparatus 2 into a predetermined compressed image data.

That is, to the moving image input board 41c in the image processing apparatus 4, is inputted the video signal of a moving image, which is generated by the video processor 22. Then, the moving image input board 41c converts the video signal of the moving image into a predetermined compressed moving image video signal data, for example, MJPEG compressed image data. The converted compressed image data is saved in the storage device 41f in the PC 41.

Note that, generally, inspection information related to endoscopy is inputted from the video processor 22 before starting the endoscopy. Based on the inputted data, the inspection information is displayed on the observation monitor 24 as character and numeral information. Furthermore, the inspection information data can be transmitted from the communication port 22a through the second communication port 41b to the image processing apparatus 4 to be recorded in the memory 41e or the storage device 41f.

In addition, the inspection information includes, for example, patient name, date of birth, sex, age, patient code, and the like.

That is, the image processing apparatus 4 is connected with the video processor 22 as needed and receives various information data from the video processor 22 to save the received data in the memory 41e or the storage device 41f.

Next, description will be made on generation of insertion shape data in the endoscope insertion shape observation apparatus 3.

The endoscope insertion shape observation apparatus 3 generates insertion shape data including three-dimensional coordinates of M-number of source coils incorporated in the insertion portion 21a of the electronic endoscope 21 for each one frame of the video signal based on the image picked up by the electronic image pickup device of the electronic endoscope 21. The endoscope insertion shape observation apparatus 3 generates an insertion portion shape image based on the insertion shape data to display the image on the monitor 33, and outputs and feeds the insertion shape data to the image processing apparatus 4.

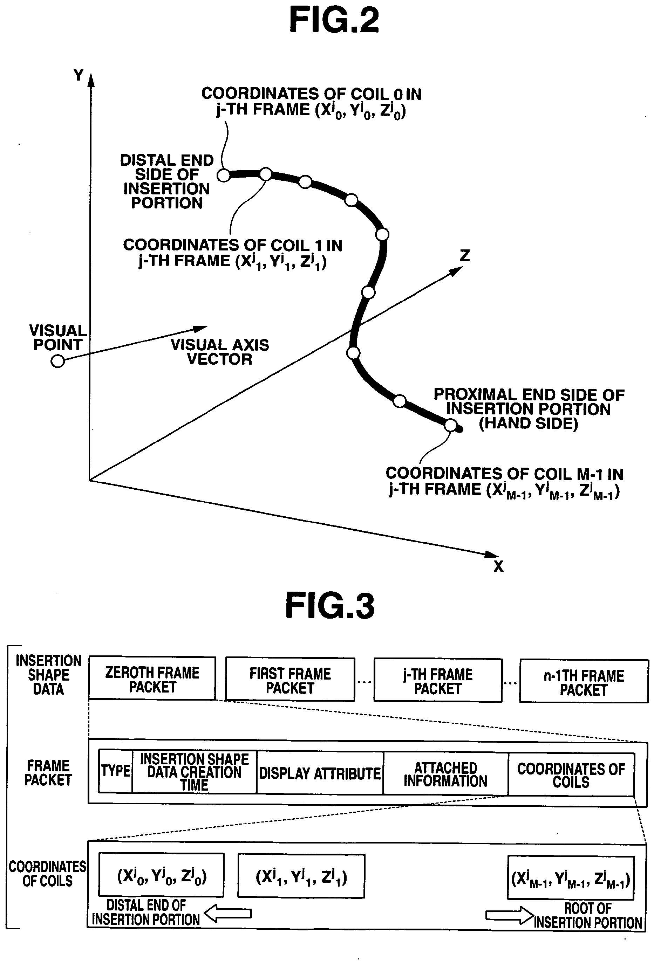

As shown in FIG. 2, M-number of source coils for estimating insertion shape are incorporated in the insertion portion 21a of the electronic endoscope 21. Then the position of each of the source coils is reference point. Coordinate system of the source coils detected by the endoscope insertion shape observation apparatus 3 is such that the three-dimensional coordinates of the m-th source coil counted from the distal end of the insertion portion 21a in j-th frame are shown by the following expression 1.

$$(X^j_m, Y^j_m, Z^j_m) \quad \cdots \quad (\text{Expression 1})$$

It is provided that m is equal to 0, 1, ..., M-1. Also, j represents the j-th frame of the video signal based on the image picked up by the electronic image pickup device.

A structure of the insertion shape data showing the coordinate system of the source coils detected by the endoscope insertion shape observation apparatus 3 is as shown in FIG. 3, and the data related to one frame is transmitted as one packet. One packet is configured of format type information, insertion shape data creation time, display attribute information, attached information, and data on coordinates of the source coils.

In the present embodiment, the format type prescribes data sizes assigned to the insertion shape data creation time, display attribute, attached information, and coil coordinates, respectively. Furthermore, prescription of the data size means to prescribe the number of data of the source coils decided for each kind of endoscope, insertion shape data creation time, accuracy of the coil coordinate, information amount included in the display attribute and attached information.

Here, the source coils are sequentially aligned from the distal end of the insertion portion 21a toward the operation portion 21c provided on the proximal end side of the insertion portion 21a. That is, the coordinate data of the source coils are three dimensional coordinates of the source coils incorporated in the insertion portion 21a of the electronic endoscope 21. Note that the coordinates of the source coils outside of the detection range by the endoscope insertion shape observation apparatus 3 are set as three-dimensional coordinates (0, 0, 0).

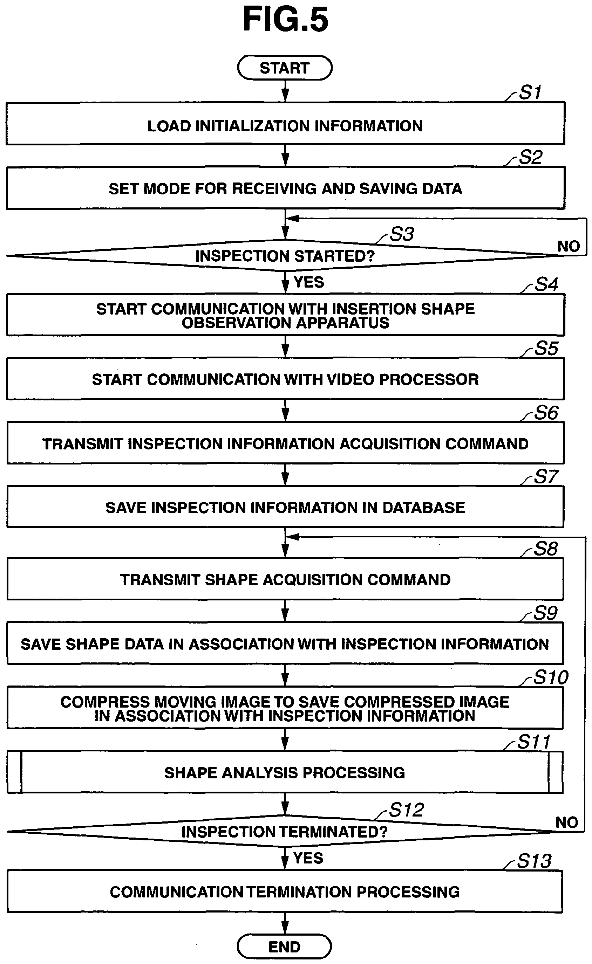

Next, a processing flow in the image processing apparatus 4 will be described using a flowchart in FIG 5. That is, description will be made on processing contents of the inspection information and the endoscope image from the video processor 22 in the endoscope apparatus 2 and the insertion shape data from the shape processing device 32 in the endoscope insertion shape observation apparatus 3.

The processing action is realized by developing and executing an application program for inspection (hereinafter application program is referred to only as application) installed in the image processing apparatus 4 by the PC 41.

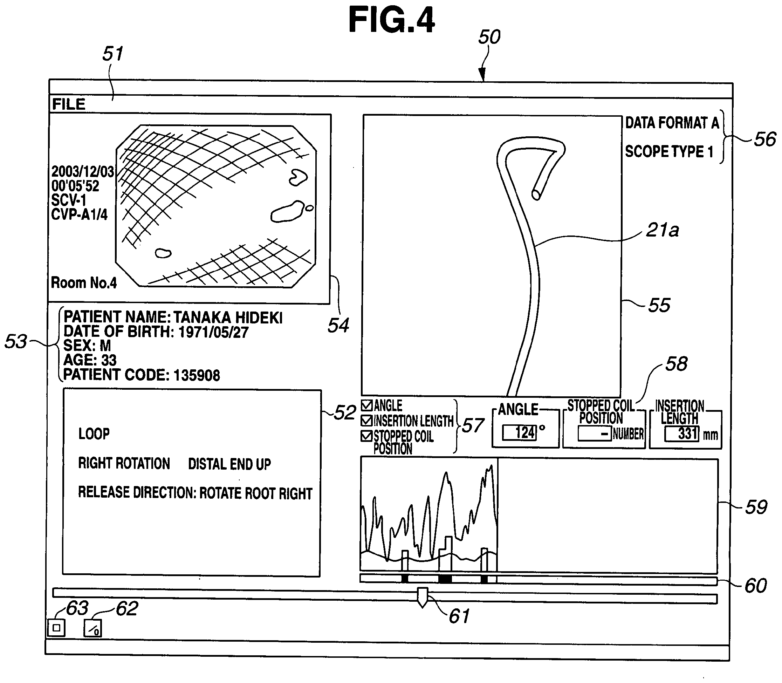

When endoscopy is started, inspection information is inputted by the video processor 22, and the application for inspection is started by the PC 41 in the image processing apparatus 4. When the application for inspection is started, an analysis window 50 shown in FIG. 4 is displayed on the display 44. Note that, FIG. 4 shows a display content during the insertion shape data is being processed.

Now, the analysis window 50 as a premise is described with reference to FIG. 4.

The analysis window 50 includes: a file menu 51; an alarm information display area 52; an inspection information display area 53; an endoscope image display area 54; an endoscope insertion shape display area 55; an attached information display area 56; display parameter check boxes 57; an analysis value display area 58; a time-series graph area 59; a time-series sub-information display area 60; a slider 61, a start button 62; and a stop button 63.

The X-axes of the time-series graph area 59 and the time-series sub-information display area 60 are time axes and in the left/right direction in FIG. 4. The time-series graph area 59 and the time-series sub-information display area 60 are areas on which a graph is created by plotting a data point every time the insertion shape data is acquired, that is, over time, and moving the plot position in the right direction in FIG. 4. A position in Y-axis direction of a data point to be plotted is decided depending on a feature amount representing the shape of the endoscope insertion portion 21a calculated by the PC 41 of the image processing apparatus 4. In the present embodiment are plotted an angle, an insertion length, and a stopped coil position as a specific position which are calculated by the endoscope image processing apparatus 4. That is, processing is performed such that the calculated specific position is visualized by a graph. Each of a scale of the Y-axis and a zero point position is individually set. Note that the Y-axis direction is the up/down direction in FIG. 4.

Options of the display parameter check boxes 57 include the angle, the insertion length, and stopped coil position in the present embodiment. By checking the check boxes, a kind of parameter to be displayed on the time-series graph area 59 is selected.

Methods of calculating the respective parameters of the angle and the insertion length are the same as those disclosed in Japanese Unexamined Patent Application Publication No. 2004-147778 as a prior art.

Next, a method for calculating the stopped coil position will be described in detail below.

When the file menu 51 in the analysis window 50 is selected, a selection window not shown for selecting insertion shape file and an image file previously recorded by the image processing apparatus 4 is displayed, and each of the files can be selected and loaded. The insertion shape file is a set file of insertion shape data generated by the endoscope insertion shape observation apparatus 3 in one inspection. The image file is a set file of the compressed image data generated by the moving image input board 41c in the image processing apparatus 4.

When the PC 41 in the image processing apparatus 4 develops and executes the application for inspection, the PC 41, as shown in FIG. 5, loads initialization information of the application from an initialization file in step S1. Note that the initialization file is stored in advance in the storage device 41f of the PC 41. Then, the PC 41 stores the information loaded from the initialization file on the memory 41e in the PC 41, and thereafter displays the analysis window 50 on the display 44.

In the present embodiment, the initialization information includes the name of the electronic endoscope 21 which is usable in the electronic endoscope system 1 and data size information for each format type of the insertion shape data. Moreover, the initialization information includes distance between each of the source coils when the insertion portion 21a of the electronic endoscope 21 is linearized, and the parameters used in the shape analysis processing. Note that, the distance between each of the source coils is referred to as inter-source coils distance information hereafter.

Moreover, the initialization information also includes display positional coordinates of the analysis window 50 on the display 44. Based on the display positional coordinates, the PC 41 displays the analysis window 50 on the display 44.

In step S2, the PC 41 sets a mode for receiving and saving the inspection information and the endoscope image data from the video processor 22 and the insertion shape data from the shape processing device 32 in the endoscope insertion shape observation apparatus 3.

Next, in step S3, the PC 41 determines whether or not the start button 62 displayed in the analysis window 50 is depressed by an operator operating the mouse 42 or the keyboard 43. The PC 41 waits until the start button 62 is depressed, and after the button is depressed, the processings after step S4 are executed.

In step S4, the PC 41 opens the first communication port 41a to start communication with the endoscope insertion shape observation apparatus 3. Then, in step S5, the PC 41 opens the second communication port 41b to start communication with the video processor 22.

In step S6, with respect to the video processor 22, the PC 41 transmits an inspection information acquisition command from the second communication port 41b to the communication port 22a of the video processor 22. The video processor 22, which has received the inspection information acquisition command, transmits the inspection information to the PC 41.

In step S7, the PC 41 records and saves in the storage device 41f the inspection information transmitted from the video processor 22 in step S6, and display the inspection information on the inspection information display area 53 in the analysis window 50.

In step S8, the PC 41 transmits an insertion shape data acquisition command from the first communication port 41a to the communication port 32a of the shape processing device 32 in the endoscope insertion shape observation apparatus 3. The shape processing device 32, which has received the insertion shape data acquisition command, starts transmission output of the insertion shape data. The transmission is continued until the communication between the PC 41 and the shape processing device 32 is terminated and the communication port 32a is closed.

In step S9, the PC 41 receives the insertion shape data transmitted and outputted from the shape processing device 32 in step S8. Then the PC 41 records and saves in the storage device 41f provided in the PC 41, as insertion shape file, the received insertion shape data in association with the inspection information that has been recorded and saved in step S7. Also, the PC 41 records the insertion shape data on the memory 41e in the PC 41 as needed.

In step S10, the PC 41 causes the moving image input board 41c to convert the moving image video signal inputted from the video processor 22 into the MJPEG compressed image data. Furthermore, the PC 41 saves in the storage device 41f in the PC 41, as an image file, the compressed image data in association with the inspection information which has been recorded and saved in step S7, and also displays the moving image inputted to the moving image input board 41c on the endoscope image display area 54 in the analysis window 50.

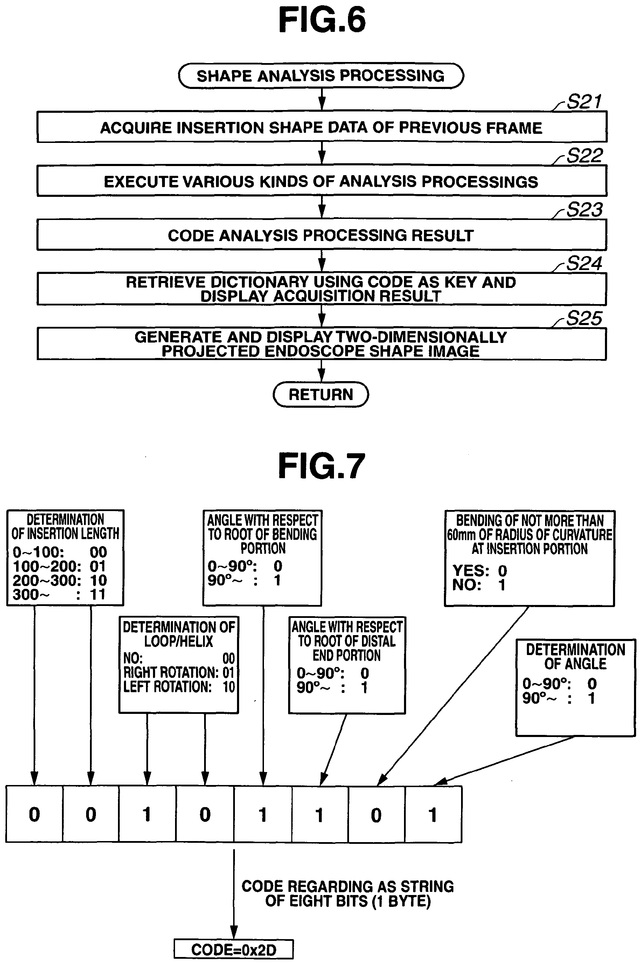

In step S11, the PC 41 executes analysis processing of each step shown in FIG. 6. When the analysis processing is terminated, the PC 41 determines whether or not the stop button 63 in the analysis window 50 has been depressed in step S12. When determining that the stop button 63 is not depressed, the PC 41 returns to step S8, and moves the slider 61 in the analysis window 50 to the right by one step.

When determining that the stop button 63 has been depressed, the PC 41, in step S13, closes the first communication port 41a and the second communication port 41b to terminate the communication of the information data between the endoscope insertion shape observation apparatus 3 and the video processor 22.

Next, description will be made on the shape analysis processing in step S11 with reference to FIG. 6.

In step S21, the PC 41 acquires the format type information of a frame packet and endoscope type information included in the attached information in the frame packet of the insertion shape data in the previous frame.

The PC 41 acquires, based on the format type of the acquired insertion shape data, the data size information of the information included in the frame packet corresponding to the format type stored on the memory 41e in step S1 in FIG. 5 and performs processing for decomposing the frame packet of the insertion shape data into each data. The name of the format type is displayed on the attached information display area 56 in the analysis window 50.

Furthermore, the PC 41 acquires the name of the electronic endoscope 21 from the attached information generated by the decomposing processing, and displays the acquired name on the attached information display area 56 in the analysis window 50. Also, the PC 41 acquires, based on the acquired name of the electronic endoscope 21, the inter-source coils distance information corresponding to the name of the electronic endoscope 21, which is stored on the memory 41e in step S1 in FIG. 5.

Then, in step S22, various analysis processings (1) to (7) described below are executed.

Processing (1): Calculation processing of insertion length (processing for counting up the source coils existing within a measurement range from the distal end)

Processing (2): Loop determination processing (processing for determining whether or not a crossing point of the insertion portion 21a is present when the insertion portion is projected in Z-axis direction (See FIG. 2))

Processing (3): Angle calculation processing (calculation processing of angle of the bending portion 21b)

Processing (4): Calculation processing of radius of curvature (calculation processing of local radius of curvature of the insertion portion 21a)

Processing (5): Calculation of angle with respect to the root (calculation processing of an angle of insertion axis direction of the source coil positioned in the vicinity of the root of the insertion portion 21a with respect to the insertion axis direction of each source coil)

Processing (6): Calculation of moving amount for one frame (calculation processing of moving amount of each source coil based on a difference between the positional coordinate of the source coil in the previous frame and that in the present frame)

Processing (7): Calculation of propulsion amount for one frame (calculation processing of a value when the moving amount is projected in an insertion axis direction at each position of the source coils)

Next, in step S23, as shown in FIG. 7, flags are generated for each of the analysis values calculated in step S22, and the flags are aligned to be regarded as a bit string, and then converted into a bytecode, whereby the analysis result is coded.

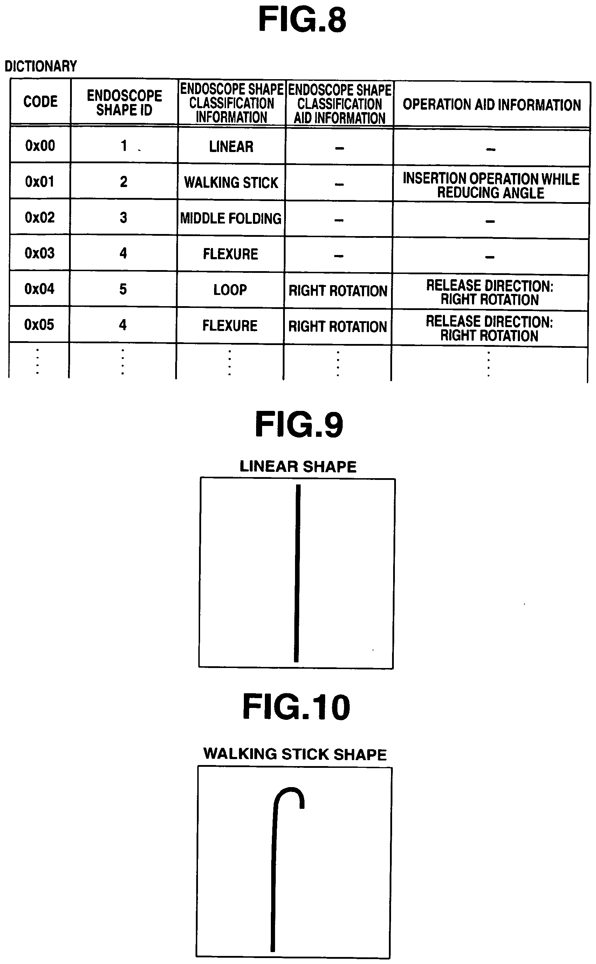

The PC 41 saves a dictionary file shown in FIG. 8 in the storage device 41f. The dictionary file manages bytecodes, and endoscope shape classification ID, endoscope shape classification information, endoscope shape classification aid information and operation aid information which are corresponding to each of the bytecodes.

In detail, the endoscope shape classification ID is created by classifying all the possible shapes that the endoscope can form and assigning an ID to each of the shapes.

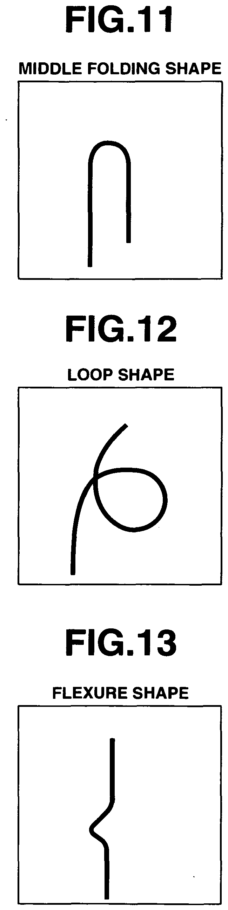

In addition, in the present embodiment, as shown in FIGS. 9 to 13, the shapes of a lower endoscope at the time of insertion can be roughly divided and classified into five patterns, for example : a linear shape (FIG. 9); a walking stick shape (FIG. 10); a middle folding shape (FIG. 11); a loop shape (FIG. 12); and a flexure shape (FIG. 13), so that character strings showing the respective patterns (linear, walking stick, middle folding, loop, and flexure) are stored in the dictionary file as endoscope shape classification information.

Furthermore, the endoscope shape classification aid information relates to a rotational direction in the insertion axis direction of the insertion portion 21a when seen from the direction from the root thereof to the distal end, in a case where the endoscope is in a loop shape or in a helical shape at the time of flexure.

Furthermore, the operation aid information is operational information of the insertion portion 21a required for eliminating factors preventing the advancement of the distal end portion such as the loop or the helical shapes, and the walking stick shape, with respect to the endoscope shapes corresponding to the respective codes. For example, when the operation aid information shows the rotational operation, the loop or helical shapes of the insertion portion 21a is released by the operator twisting the root of the insertion portion 21a in the direction shown by the operation aid information, and the shape of the insertion portion 21a is changed so as to be in a straight shape.

Then, in step S24, the dictionary file is retrieved using the bytecode generated in step S23 as a key, and acquired result is displayed on the alarm information display area 52 in the analysis window 50 (See FIG. 4). Specifically, information falling under the bytecode obtained in step S23 is retrieved and acquired from the dictionary file, and character information (endoscope shape classification information, endoscope shape classification aid information, and operation aid information) is displayed on the alarm information display area 52 in the analysis window 50. Note that the display processing is not performed when there is no information falling under the bytecode.

After that, in step S25, a two-dimensionally projected endoscope image related to the insertion shape data is generated and displayed on the endoscope insertion shape display area 55 in the analysis window 50, and the processing proceeds to step S12.

Thus, in the present embodiment, every shape of the endoscope insertion portion is coded and the endoscope shape pattern and operation aid information which are corresponding to each of the codes are displayed, so that an integrated shape determination processing system based on a plurality of determination conditions can be easily constructed, thereby improving the accuracy of the aid information presented for the operator.

That is, it is possible to classify the shapes of the endoscope insertion portion into patterns based on the determination result obtained by integrating the analysis results and provide the information corresponding to each of the patterns. Therefore, it is possible to stably perform a shape analysis and to analyze the endoscope insertion state with the whole shape of the insertion portion.

In addition, the information corresponding to each of the codes is displayed by retrieving and acquiring from the dictionary file, so that the display contents can be changed only by editing the dictionary file without changing (compiling and rebuilding) the program.

(Second Embodiment)

The second embodiment is almost the same as the first embodiment, so that only the different points are described, and the same components are attached with the same reference numerals and description thereof will be omitted.

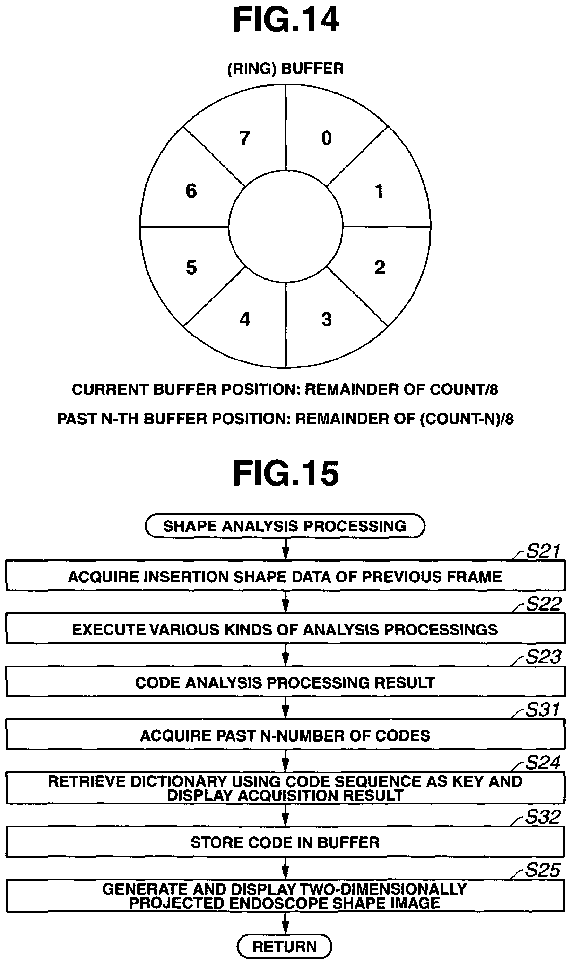

In the present embodiment, in the memory 41e, a ring buffer 90 of size eight as shown in FIG. 14 is provided, for example.

The insertion shape data is related to data acquisition count "Count" from the start of the inspection, so that, in the present embodiment, storage and acquirement of insertion shape data of past N-number of times (N<8) are realized by setting the remainder of Count/8 as the storage position of the ring buffer 90. Other configurations are the same as those in the first embodiment.

In the present embodiment, a part of the shape analysis processing in step S11 is different from that in the first embodiment, so that different points are described with reference to FIGS. 15 to 17.

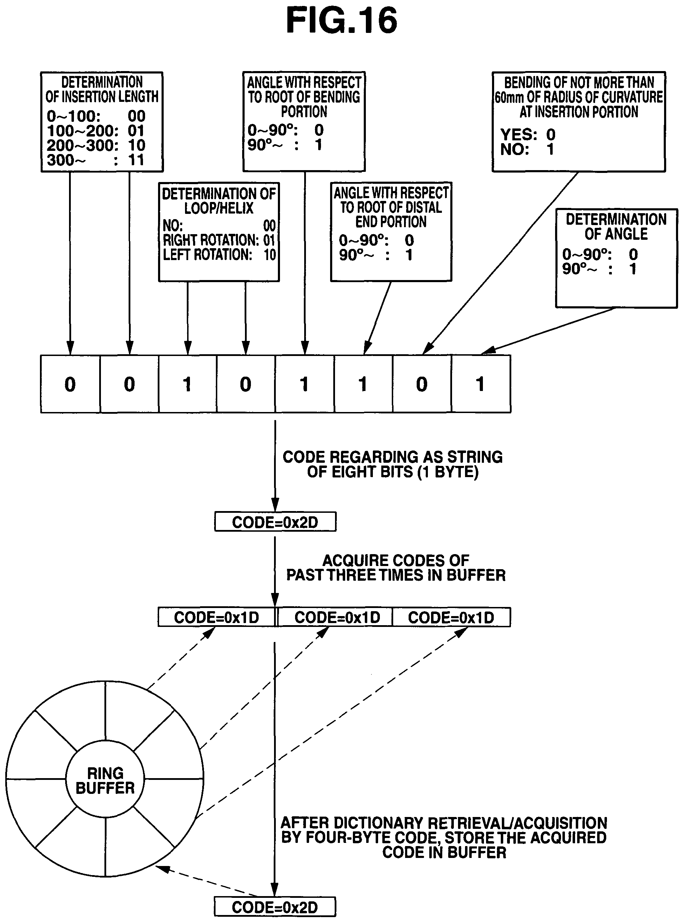

In the shape analysis processing in the present embodiment, as shown in FIGS. 15 and 16, after the processings in steps S21 to S23 described in the first embodiment, for example the storage information (insertion shape data) of past three times stored in the ring buffer 90 is acquired in step S31. The position of the ring buffer 90 is calculated from the remainder of each of (Count-1)/8, (Count-2)/8, and (Count-3)/8 with respect to the current data acquisition count "Count". Then the four-byte code is generated by sequentially combining the acquired storage information of past three times and the bytecodes obtained in step S23.

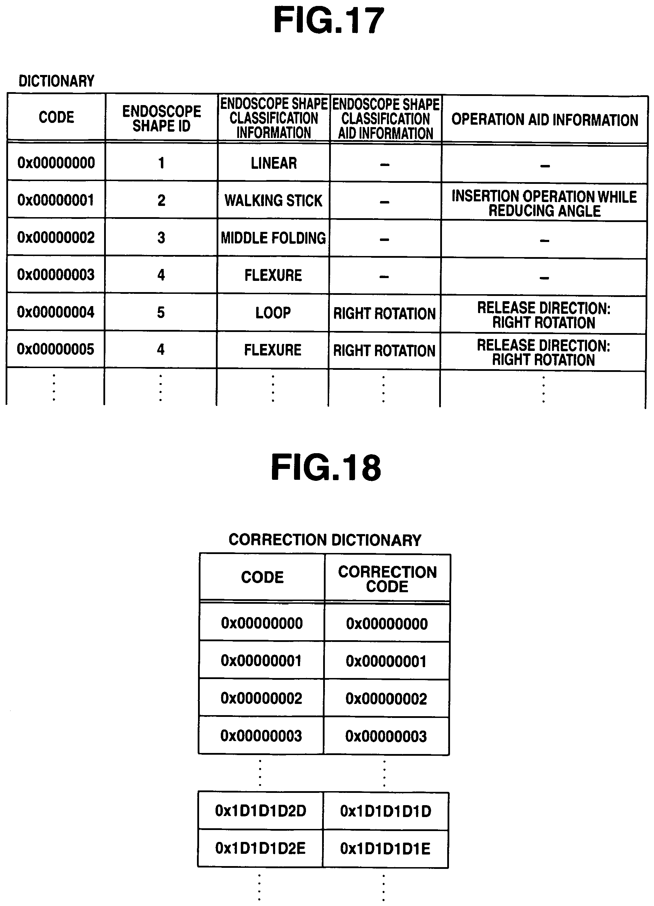

In the present embodiment, the PC 41 saves the dictionary file shown in FIG. 17 in the storage device 41f. The dictionary file manages the four-byte codes and endoscope shape classification ID, endoscope shape classification information, endoscope shape classification aid information and operation aid information which are corresponding to each of the four-byte codes.

Then, in step S24, information falling under the four-byte code obtained in step S31 is retrieved and acquired from the dictionary file, and character information (endoscope shape classification ID, endoscope shape classification information, endoscope shape classification aid information, and operation aid information) is displayed on the alarm information display area 52 in the analysis window 50. Note that the display processing is not performed when there is no information falling under the bytecode.

Next, in step S32, the bytecode obtained in step S23 is stored at the appropriate position in the ring buffer 90 in the memory 41e of the PC 41.

After that, same as in the first embodiment, in step S25, a two-dimensionally projected endoscope image related to the insertion shape data is generated and displayed on the endoscope insertion shape display area 55 in the analysis window 50, and thereafter the processing proceeds to step S12.

Note that, more simply, the operation aid information to be displayed may be decided by storing in the memory 41e the previous processing result of the insertion shape data and by performing comparison processing between this time's processing result of the insertion shape data and the previous one (for example, compare whether or not the previous processing result is the same as this time's processing result).

In addition, a code correction dictionary shown in FIG. 18 may be stored in the storage device 41f of the PC 41, and the four-byte codes may be corrected using the code correction dictionary in step S31.

Thus, in the present embodiment, same as in the first embodiment, every shape of the endoscope insertion portion is coded, and the endoscope shape pattern and the operation aid information, which are corresponding to the four-byte code generated by combining the bytecode and the previously acquired bytecode, are displayed. Therefore, erroneous processing due to disturbance is prevented, and determination with respect to a series of endoscope operation becomes possible. As a result, the accuracy of the operation aid information presented for the operator is improved.

In addition, the information corresponding to each of the four-byte codes is displayed by retrieving and acquiring from the dictionary file, so that the display contents can be changed only by editing the dictionary file without changing (compiling and rebuilding) the program.

(Third Embodiment)

The third embodiment is almost the same as the first embodiment, so that only the different points are described, and the same components are attached with the same reference numerals and description thereof will be omitted.

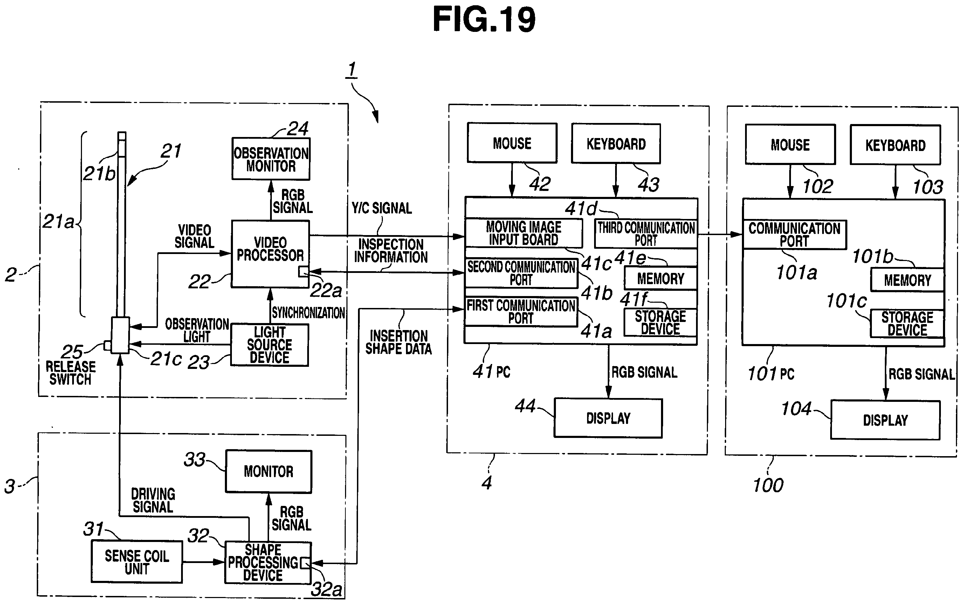

As shown in FIG. 19, the PC 41 of the image processing apparatus 4 in the present embodiment includes a third communication port 41d communicable with an image filing apparatus 100 as an external apparatus, for example.

The image filing apparatus 100 includes a PC 101 to manage/record image data, a mouse 102, a keyboard 103, and a display 104 which are connectable to the PC 101. The PC 101 of the image filing apparatus 100 includes a communication port 101a communicable with the PC 41 of the image processing apparatus 4, a memory 101b, and a storage device 101c.

FIG. 19 is a block diagram showing configurations of a processing block of an insertion shape analysis application 151, an insertion shape data recording application 151a, an insertion shape display application 151b, and an insertion shape data management application 152, all of which are running programs run by the PC 41 of the image processing apparatus 4, and a memory block of the memory 41e used by each of the applications.

The memory 41e includes a memory for insertion shape analysis application 141a used by the insertion shape analysis application 151, a memory for insertion shape data management application 141b used by the insertion shape data management application 152, and a shared memory 141c shared by the insertion shape analysis application 151 and the insertion shape data management application 152. The shared memory 141c is accessible either from the insertion shape analysis application 151 and the insertion shape data management application 152.

In addition, the insertion shape data management application 152 includes a first thread composed of an insertion shape data acquisition block 161 and a message transmission block 162, a second thread composed of an insertion shape data transmission block 163, and a third thread composed of an output destination management block 164 and an output destination registration block 165.

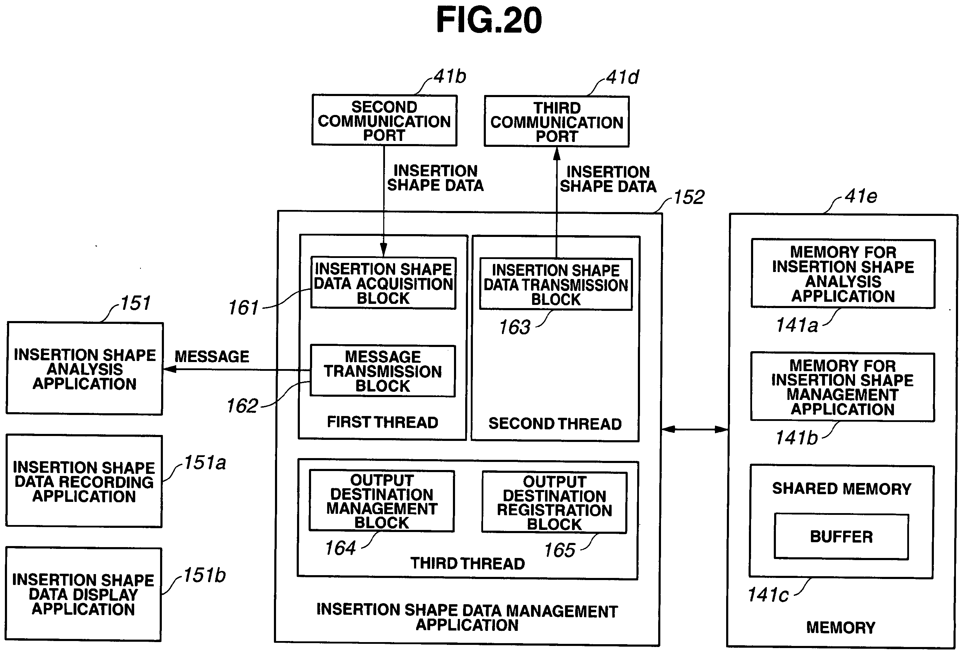

The working of the present embodiment thus configured is described with reference to FIGS. 20 to 23.

The output destination registration block 165 in the third thread displays a registration window 171 shown in FIG. 20 on the display 44, and stores presence or absence of a check in check boxes 172 in the registration window 171 in the area of the memory for insertion shape data management application 141b in the memory 41e.

The insertion shape transmission block 163 in the second thread confirms, through the output destination management block 164 in the third thread, whether or not the content showing that the image filing apparatus 100 is checked as the transmission destination is stored in the area of the memory for insertion shape data management application 141b in the memory 41e.

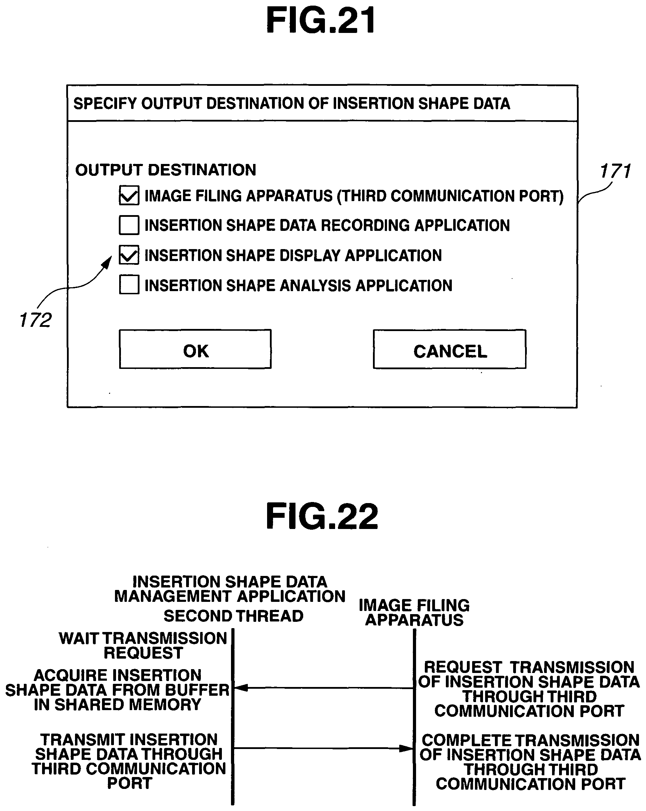

After that, in a case where the image filing apparatus 100 is specified as the transmission destination, the insertion shape transmission block 163 in the second thread performs transmission/reception processing with the image filing apparatus 100 through the third communication port 41d according to the flow shown in FIG. 21.

When receiving transmission request from the image filing apparatus 100, the insertion shape data transmission block 163 in the second thread loads the insertion shape data stored in the buffer in the shared memory 141c, to transmit the insertion shape data to the image filing apparatus 100 using the third communication port 41d. When the transmission is completed, the insertion shape data transmission block 163 moves on to a waiting state of the transmission request from the image filing apparatus 100 to repeat the same processing.

The insertion shape data acquisition block 161 in the first thread confirms, through the output destination management block 164 in the third thread, whether or not the content showing that the insertion shape data recording application 151a or the insertion shape display application 151b, or the insertion shape analysis application 151 is checked as the transmission destination is stored in the area of the memory for insertion shape data management application 141b in the memory 41e.

The first thread performs transmission/reception with the endoscope insertion shape observation apparatus 3 through the communication port 32a according to the flow shown in FIG. 22. In the present embodiment, description will be made assuming that the insertion shape analysis application 151 is checked as the transmission destination.

The insertion shape data acquisition block 161 requests the endoscope insertion shape observation apparatus 3 to transmit the insertion shape data through the communication port 2.

The endoscope insertion shape observation apparatus 3 transmits the insertion shape data through the communication port 32a, and the insertion shape data acquisition block 161 receives the insertion shape data to write the data in the buffer in the shared memory 141c.

Subsequently, the message transmission block 162 retrieves/acquires a window handle of the insertion shape analysis application 151 from the OS (Operating System), and transmits a message to the window handle when acquiring a valid window handle. As an argument of the message, position of the buffer in the shared memory 141c is specified.

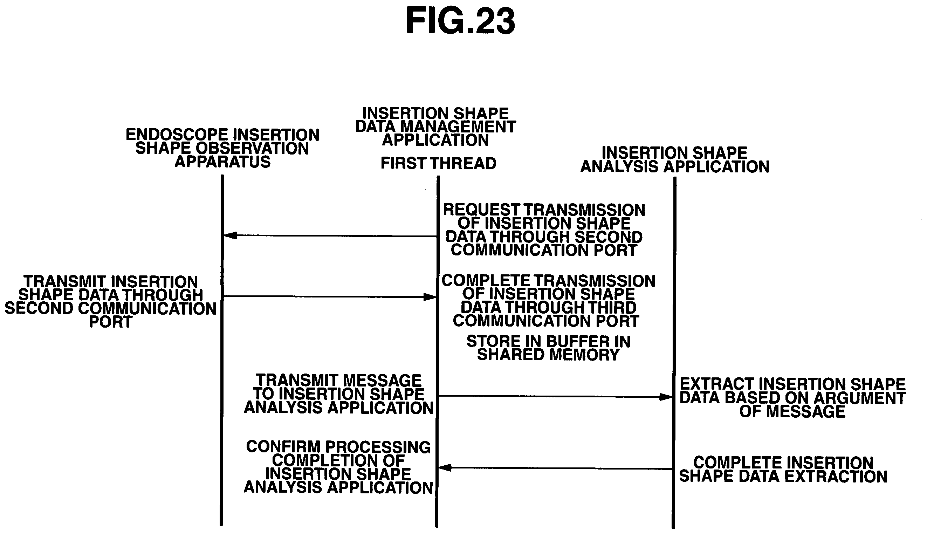

As shown in FIG. 23, the insertion shape analysis application 151 accesses the buffer in the shared memory 141c based on the argument of the message to acquire the insertion shape data in step S41. This timing is equivalent to step S8 in FIG. 4 described in the first embodiment.

The insertion shape analysis application 151 copies the acquired insertion shape data in the memory for insertion shape data management application 141b in the memory 41e to convey the confirmation showing processing completion to the insertion shape data acquisition block 161.

After transmitting the message, the insertion shape data acquisition block 161 continues to wait for confirmation to be conveyed from the insertion shape analysis application 151, and repeats the same processing at the time of receiving the confirmation conveyance.

Note that exclusive processing at the time of loading and writing into the shared memory 141c is realized using an atomic structure of loading and writing in a memory prepared by the OS.

Thus, in the present embodiment, in addition to the effect of the first embodiment, since it is easy to run in parallel a plurality of processing modules having divided functions, because the insertion shape data is used in a shared manner among a plurality of applications and a plurality of apparatuses, it is facilitated to construct a system with a configuration as needed and enable the cost of system development to be reduced.

(Fourth Embodiment)

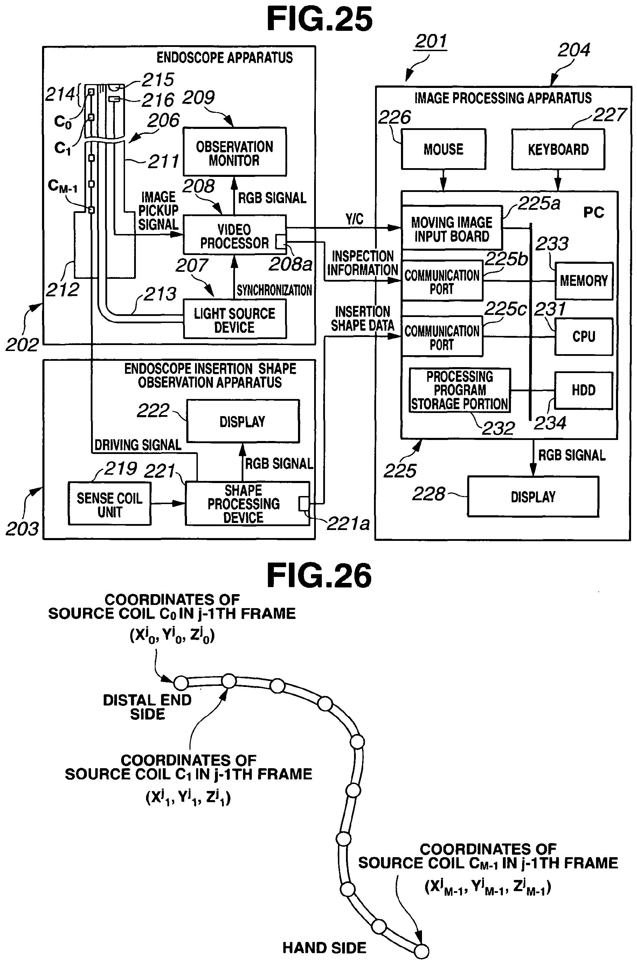

As shown in FIG. 25, an in-vivo insertion monitoring system 201 provided with the fourth embodiment of the present invention includes: an endoscope apparatus 202 for performing endoscopy; an endoscope insertion shape observation apparatus 203 for observing an endoscope insertion shape; and an image processing apparatus 204 configuring the fourth embodiment of an insertion monitoring apparatus for analyzing the endoscope insertion shape data (abbreviated as insertion shape data) generated by the endoscope insertion shape observation apparatus 203 to assist or support the endoscopy.

The endoscope apparatus 202 includes: an electronic endoscope 206 to be inserted into a body cavity such as large intestine; a light source device 207 for supplying illumination light to the electronic endoscope 206; a video processor 208 as a signal processing device for performing signal processing on an image pickup device 216 such as CCD incorporated into the electronic endoscope 6; and an observation monitor 209, to which the video signal generated by the video processor 208 is inputted, for displaying the image in the body cavity picked up by the image pickup device 216 as an endoscope image.

The electronic endoscope 206 includes an elongated insertion portion 211 to be inserted into a patient's body cavity and an operation portion 212 provided at a rear end of the insertion portion 211. Inside the insertion portion 211, the light guide 213 for transmitting the illumination light is inserted. The light guide 213 has a rear end connected to the light source device 207, and transmits the illumination light supplied from the light source device 207 to emit (the transmitted illumination light) from an illumination window provided at a distal end portion 214 of the insertion portion 211.

Note that the insertion portion 211 has a freely bendable bending portion at a rear end of the distal end portion 214 and is capable of bending the bending portion by operating an bending operation knob and the like, not shown, provided in the operation portion 212.

The distal end portion 214 has an objective lens 215 mounted to the observation window provided adjacently to the illumination window. An optical image is formed by the objective lens 215 on an image pickup surface of the image pickup device 216 such as a charge coupled device (abbreviated as CCD) disposed at an image forming position of the objective lens 215.

The image pickup device 216, which is connected with the video processor 208 via a signal line, outputs to the video processor 208 an image pickup signal obtained by photoelectrically converting the optical image.

The video processor 208 performs a signal processing for generating a video signal on the image pickup signal outputted from the image pickup device 216. Then, the video processor 208 outputs to the observation monitor 209 the generated video signal, for example, RGB signal. As a result, on the display surface of the observation monitor 209, the image picked up by the image pickup device 216 is displayed.

Note that, when performing frame sequential illumination by R, G, B illumination lights, the light source device 207 outputs to the video processor 208 synchronization signals synchronized with each of the illumination periods, and the video processor 208 performs signal processing synchronously with the synchronization signals.

Furthermore, the electronic endoscope 206 has a switch, not shown, for giving release instruction and the like provided on the operation portion 212, and is capable of controlling the action of the video processor 208 by operating the switch.

In addition, the present embodiment includes a detection function for detecting an insertion position and an insertion shape of the insertion portion 211 to be inserted in a body cavity. Specifically, the electronic endoscope 206 includes inside the insertion portion 211 in the longitudinal direction thereof a plurality of source coils C0, C1, ..., CM-1 (abbreviated as C0 to CM-1) disposed at a predetermined interval, and the source coils C0 to CM-1 generate magnetic fields around thereof when being applied with a driving signal.

Then the magnetic fields generated by the source coils C0 to CM-1 are detected by a sense coil unit 219 incorporating a plurality of sense coils, which is provided in the endoscope insertion shape observation apparatus 203.

That is, the endoscope insertion shape observation apparatus 203 includes: the sense coil unit 219 for detecting the magnetic fields generated by the source coils C0 to CM-1 provided in the electronic endoscope 206; a shape processing device 221 for estimating the shape of the insertion portion 211 (referred to as insertion shape) based on detection signals of the magnetic fields detected by the sense coil unit 219; and a display 222 for displaying the insertion shape estimated by the shape processing device 221.

The sense coil unit 219 is disposed, for example, around an inspection bed on which a patient lies, to detect the magnetic fields generated by the source coils C0 to CM-1 and output the detected detection signals to the shape processing device 221.

The shape processing device 221 calculates each positional coordinate data of each of the source coils C0 to CM-1 based on the detection signals, to estimate the insertion shape of the insertion portion 211 from the calculated positional coordinate data.

The shape processing device 221 generates the video signal of the estimated insertion shape of the insertion portion 211 to output the generated video signal, for example, the RGB signal to the display 222. Then, the insertion shape is displayed on the display screen of the display 222. It becomes easy for the operator to more smoothly perform the insertion operation by observing the insertion shape.

In addition, during the endoscopy, the shape processing device 221 continuously generates insertion shape data such as three-dimensional coordinate information showing the insertion shape and shape display attributes to output the generated insertion shape data to the image processing apparatus 204 through a communication port 221a. The shape processing device 221 can output to the image processing apparatus 204 only the insertion shape data of when the release switch is operated.

Note that the endoscope insertion shape observation apparatus 203 can change the shape display attributes such as rotation angle, magnification/reduction rate, and the like of the image of insertion shape generated by the shape detection processing by the shape processing device 221 and displayed on the display 222, by inputting instruction from an operation panel not shown or the like.

Note that the video processor 208 has a function for inputting inspection information related to the endoscopy, though not shown, so that the inspection information inputted to the video processor 208 is transmitted also to the image processing apparatus 204 through a communication port 208a.

The image processing apparatus 204, with respect to the inputted insertion shape data, performs analysis processing on a response action state of the insertion portion 211 actually inserted into a body cavity with respect to the endoscope operation such as insertion operation, and determines whether or not the response action state is a predetermined state to be notified to the operator. When the response action state is the predetermined state, the image processing apparatus 204 generates insertion aid information.

To this end, the image processing apparatus 204 includes: a personal computer (hereinafter called only as PC) 225 for performing analysis processing to generate the insertion aid information for assisting or supporting the operator; a mouse 226 and a keyboard 227 for inputting various instructions to the PC 225; and a display 228 as a display device for reproducing or displaying the insertion aid information and the like generated by the analysis processing by the PC 225.

The PC 225 includes: a communication port 225a for loading the insertion shape data outputted from the communication port 221a of the shape processing device 221 in the endoscope insertion shape observation apparatus 203; a communication port 225b for loading the endoscopy information outputted from the communication port 208a of the video processor 208 in the endoscope apparatus 202; an moving image input board 225c for converting the video signal of the moving image picked up by the image pickup device 216 in the electronic endoscope 206 and generated by the video processor 208 into predetermined compressed image data; a CPU 231 for performing image processing; a processing program storage portion 232 in which a processing program for performing image processing by the CPU 231 is stored; a memory 233 for temporarily storing the data and the like to be processed by the CPU 231; and a hard disk (HDD) 234 as a storage device for storing the processed image data and the like. The CPU 231 and the like are connected with one another by buses.

To the moving image input board 225c in the image processing apparatus 204 is inputted the video signal of the moving image generated by the video processor 208, the Y/C signal, for example. The moving image input board 225c converts the video signal of the moving image into a predetermined video signal data of the compressed moving image, for example, MJPEG compressed image data to save the data in the hard disk 234 and the like in the PC 225.

Note that, before starting an endoscopy, inspection information related to the endoscopy is inputted from the video processor 208 and displayed on the observation monitor 209 in forms of characters and numbers based on the inputted inspection information data, and the inspection information data can be transmitted from the communication port 208a via the communication port 225b in the image processing apparatus 204 to the PC 225 and recorded therein.

Note that the inspection information includes, for example, patient name, date of birth, sex, age, patient code, and inspection date, and the like.

That is, the image processing apparatus 204 is connected to the video processor 208 as needed, and receives and saves various kinds of information data from the video processor 208.

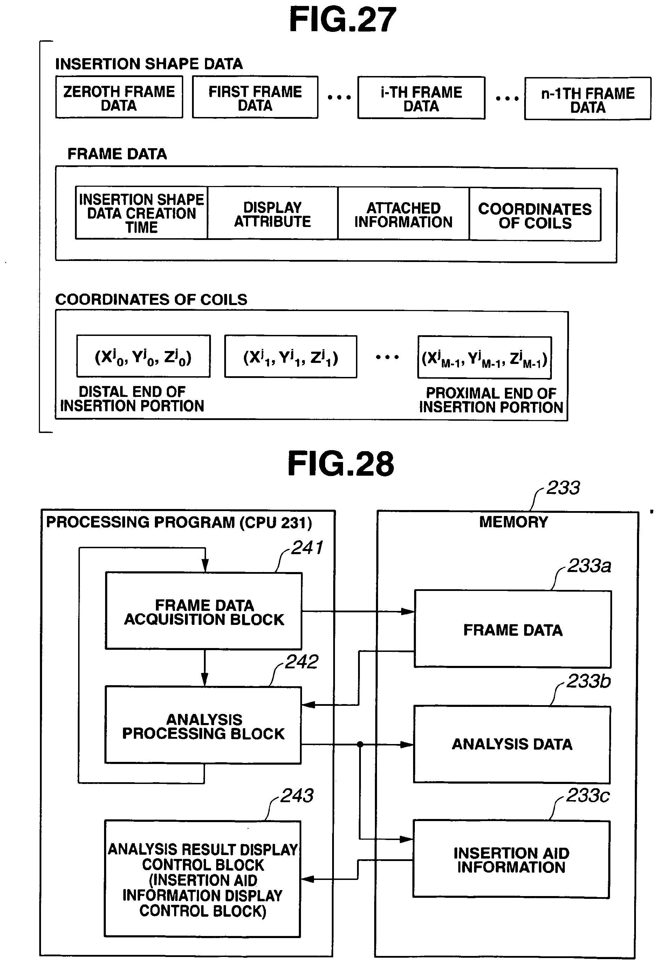

Description will be made on generation of insertion shape data by the endoscope insertion shape observation apparatus 203 in the in-vivo insertion monitoring system 201 thus configured, with reference to FIGS. 26 and 27.

The shape processing device 221 of the endoscope insertion shape observation apparatus 203 generates insertion shape data including the three-dimensional coordinates of M-number of source coils C0 to CM-1 incorporated in the insertion portion 211 of the electronic endoscope 206 for each one frame of the image pickup signal of the image picked up by the image pickup device 216 of the electronic endoscope 206. Furthermore, the shape processing device 221 generates an image of insertion shape based on the insertion shape data to display the generated image on the display 222, and outputs the insertion shape data to the image processing apparatus 204.

The coordinate systems of the source coils C0 to CM-1 detected by the endoscope insertion shape observation apparatus 203 are shown in FIG. 26, when taking a case of a j-lth frame (note that, as shown in FIG. 27, j is assumed such that the first frame is the zeroth frame) as an example.

As shown in FIG. 26, the three-dimensional coordinates of the source coil Ci which is the i-1th (note that i=0, 1, ..., M-1) source coil from the distal end side of the insertion portion 11 are expressed by $(X^j_i, Y^j_i, Z^j_i)$.

A structure of the insertion shape data including the data of the coordinate systems of the source coils C0 to CM-1 detected by the endoscope insertion shape observation apparatus 203 is shown in FIG. 27. The insertion shape data is sequentially transmitted to the image processing apparatus 204, in an image pick-up order of the frames, the frame data related to each of the frames (that is, the zeroth frame data, the first frame data,...) as one packet. Each of the frame data transmitted as the packet includes data such as insertion shape data creation time, the display attributes, the attached information, (source) coil coordinates, and the like.

In addition, the coil coordinate data respectively show the three dimensional coordinates of the source coils C0 to CM-1 which are disposed in sequence from the distal end of the insertion portion 211 to the operation portion 212 on the proximal end (hand side) of the insertion portion, as shown in FIG. 26. Note that, it is assumed that the coordinates of the source coils outside the detection range of the endoscope insertion shape observation apparatus 203 are set to predetermined constant numbers, for example, so as to show the coordinates are outside of the detection range.

Next, with reference to FIGS. 28 to 30, description will be made on the processings from the acquirement of inspection information and the endoscope image from the video processor 208 of the endoscope apparatus 202 in the image processing apparatus 204 and the insertion shape data from the shape processing device 221 of the endoscope insertion shape observation apparatus 203 to the generation of insertion aid information, and also working and the like of monitoring of endoscopy in the large intestine by inserting the electronic endoscope 206 into the large intestine, for example.

When the endoscopy is started, in the image processing apparatus 204, the CPU 231 configuring the PC 225 starts processings according to a processing program stored in the processing program storage portion 232.

The processing function block executed by the CPU 231, as shown in FIG. 28, includes: a frame data acquisition block 241 for acquiring frame data to store the acquired frame data in the memory 233; an analysis processing block 242 for performing analysis processing on the frame data stored in the memory 233 to store analysis data 233b and generated insertion aid information 233c in the memory 233; and an analysis result display control block 243 for displaying an analysis result and , controlling the display (or display characteristics) of the insertion aid information 233c.

As shown in FIG. 28, the frame data acquisition block 241 and the analysis processing block 242 repeatedly performs processings like a loop. The analysis processing block 242, as an analysis result, performs processing of determining a condition corresponding to a predetermined action state. In a case where the predetermined response action state falls under the determined condition, the analysis processing block 242 generates insertion aid information. Furthermore, the analysis result display control block 243 performs display control for controlling the display and the display stop (deletion) of the insertion aid information 233c according to elapsed-time information set in advance.

The frame data acquisition block 241 stores on the memory 233 the frame data transmitted from the endoscope insertion shape observation apparatus 203 as shown in FIG. 28, and save the frame data in the hard disk 234 as shown in FIG. 25.

The analysis processing block 242, by using the frame data 233a on the memory 233, calculates data to examine the response action state of the insertion portion 211 (with respect to the insertion operation) such as the direction of the insertion portion 211 in each position of the source coils and moving amounts of the source coils in one frame before the present frame. The analysis processing block 242 stores the calculated data on the memory 233 as the analysis data 233b.

In addition, the analysis processing block 242 generates the analysis data 233b from the frame data 233a on the memory 233, and performs analysis processing for generating the insertion aid information 233c by using the frame data 233a (and analysis data 233b as needed) in order to display, as the insertion aid information, the information related to the response action state where the insertion portion 211 to be inserted into a body cavity is not smoothly or appropriately inserted with respect to the insertion operation by the operator.

In this case, in a case where the analysis data 233b indicates the response action state satisfying a predetermined condition, specifically, the condition in which, when the operator performs operation to push the insertion portion 211 at the proximal end side thereof into the body cavity, the distal end side of the insertion portion 211 barely moves, in other words, the insertion of the insertion portion 211 cannot performed smoothly, the analysis processing block 242 generates the insertion aid information 233c to store the generated insertion aid information on the memory 233. In this case, if the previous insertion aid information 233c is stored on the memory 233, the contents of information are updated.

Here, an additional description will be made on the predetermined condition related to the insertion aid information 233c.

When the ratio of a moving amount M0 of the source coil C0 at the distal-most end position of the insertion portion 211 with respect to an axial direction of the insertion portion 211 to a moving amount Mn of, for example, the source coil CM-1 at the hand side position of the insertion portion 211 with respect to the axial direction of the insertion portion 211, that is, the ratio M0/Mn satisfies the condition in which the ratio is smaller than a threshold value (here 0.1), (that is, M0/Mn < 0.1), the insertion aid information 233c is generated.

The insertion aid information 233c generated when the above-described condition is satisfied is composed of a character string information "distal end stop" and the insertion shape data creation time T0 in the frame data used for the condition determination in the present embodiment.

Note that, when the above-described condition is satisfied, the state indicates that the distal end portion 214 of the insertion portion 211 almost stops even though the operator is performing the insertion operation at the hand side of the insertion portion 211, accordingly, the distal end portion 214 does not advance even if the operator further pushes the hand side of the insertion portion.

On the other hand, the analysis result display control block 243 is a processing block executed at certain time intervals independently from the loop processings of the frame data acquisition block 241 and the analysis processing block 242.

The analysis result display control block acquires the insertion aid information on the memory 233, and, when difference between the current time Tn and the insertion shape data creation time T0 corresponding to the generation of the insertion aid information 233c is smaller than a predetermined threshold value Tt set in advance (Tn-T0<Tt), displays the character string information of the insertion aid information 233c on the display 228 of the image processing apparatus 204 as "distal end stop", for example. That is, the analysis result display control block maintains displaying the character string information of the insertion aid information 233c when the time elapsed from the insertion shape data creation time T0 used in generating the insertion aid information 233c is smaller than the threshold value Tt as the predetermined time.

Note that, though a default value is set in advance, the threshold value Tt can be changed to be set to a value which a user such as an operator considers more appropriate through the keyboard 227, for example. The threshold value Tt may be changed to be set in association with ID information of a user so as to be set to a different value for each user.

That is, there is a possible situation such that rough time interval at which an operator performing endoscopy observes the display 228 on which the insertion aid information is displayed is different for each user. Therefore, it may be configured such that the threshold value Tt can be set for each operator so that each operator can confirm the display (of the character string information) in the insertion aid information 233c more appropriately.

In addition, in a case of Tn-T0>Tt, the analysis result display control block deletes the character string information displayed on the display 228 of the image processing apparatus 204.

Thus, in the present embodiment, when it is determined to satisfy the condition corresponding to the response action state in which the relative moving amount of the distal end side of the insertion portion 211 with respect to the insertion operation at the proximal end side of the insertion portion 211 is small enough and the distal end side barely moves, the insertion aid information 233c is generated. In addition, the display of the insertion aid information 233c is maintained from the insertion shape data creation time T0 used for the generation of the insertion aid information 233c to the time at which the operator can easily confirm the display.

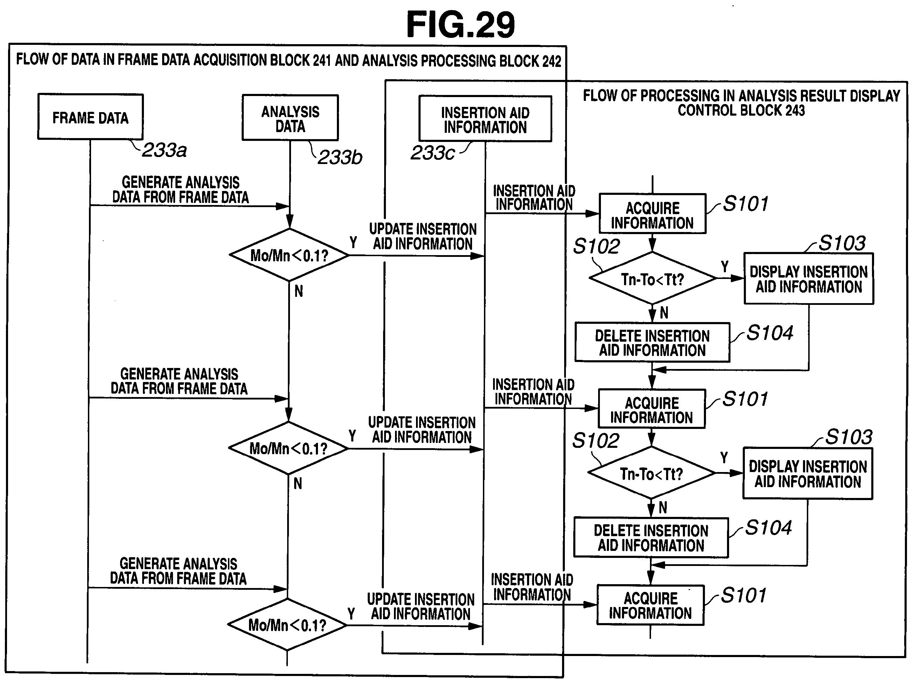

Next, description will be made on the flow of data processed in the frame data acquisition block 241 and the analysis processing block 242 and the processing flow in the analysis result display control block 243, with reference to FIG. 29.

When performing endoscopy in the body cavity, for example, in the large intestine, the operator inserts the insertion portion 211 of the electronic endoscope 206 shown in FIG. 25 from the anus of the patient into the large intestine. In this case, the operator grasps the proximal end side of the insertion portion 211 and inserts the insertion portion 211 in order from the distal end portion 214 side thereof into a deep portion side of the large intestine.

The image pickup signal of the image picked up by the image pickup device 216 provided in the distal end portion 214 of the insertion portion 211 of the electronic endoscope 206 is subjected to signal processing in the video processor 208, and then a video signal is generated and an endoscope image is displayed on the observation monitor 209.

Each position of the source coils C0 to CM-1 which are disposed in the longitudinal direction of the insertion portion 211 is detected by the shape processing device 221 based on the detection signal from the sense coil unit 219, and the insertion shape is displayed on the display 222 of the endoscope insertion shape observation apparatus 203.

The frame data including the positional information of each of the (source) coils is transmitted from the shape processing device 221 to the PC 225 of the image processing apparatus 204. Then, as shown in FIG. 28, the frame data acquisition block 241 in the CPU 231 (in the PC 225) stores the frame data 233a on the memory 233.

In addition, as shown in FIG. 29, the analysis processing block 242 performs analysis processing with respect to the frame data 233a (stored on the memory 233) to generate the analysis data 233b from the frame data 233a and stores the generated analysis data on the memory 233.

Furthermore, as shown in FIG. 29, the analysis processing block 242 performs determination processing with respect to the analysis data 233b as to whether or not the analysis data satisfies a predetermined condition (M0/Mn<0.1 in the present embodiment). When the analysis data satisfies the condition, the analysis processing block 242 generates the insertion aid information 233c to store (overwrite) the generated insertion aid information on the memory 233. In this case, if the old insertion aid information 233c has already been stored on the memory 233, the old information is updated. The above processings are repeatedly executed.

Furthermore, as shown in FIG. 29, the analysis result display control block 243 performs processing for acquiring the insertion aid information 233c on the memory 233 at certain time intervals (step S101) and also acquires the current time Tn.

In addition, in the next step S102, the analysis result display control block 243 performs determination processing for determining whether or not the difference between the current time Tn and the insertion shape data creation time T0 in the insertion aid information is smaller than the predetermined threshold value Tt (Tn-T0<Tt).

Then, as shown in step S103, when determining that the condition of Tn-T0<Tt is satisfied, the analysis result display control block 243 displays the character string information of the insertion aid information 233c on the display 228 of the image processing apparatus 204. After that, the analysis result display control block 243 prepares for the acquisition processing of the next insertion aid information 233c.

On the other hand, if the determination processing in step S102 determines that the difference between the current time Tn and the insertion shape data creation time T0 in the insertion aid information 233c is equal to or larger than a predetermined threshold value Tt (Tn-T0≥Tt), the analysis result display control block 243 deletes the character string information of the insertion aid information 233c displayed on the display 228 of the image processing apparatus 204, as shown in step S104. Then, the analysis result display control block 243 prepares for the acquisition processing of the next insertion aid information 233c.

By observing the character string information of the insertion aid information 233c displayed on the display 228 of the image processing apparatus 204, the operator can confirm whether or not the analysis data shows a predetermined response action state which the operator wants to know, even if the observation timing is later than the timing when the insertion aid information 233c was generated.

Thus, the analysis result display control block 243 repeats the processings in step S101 to S104 at certain time intervals.

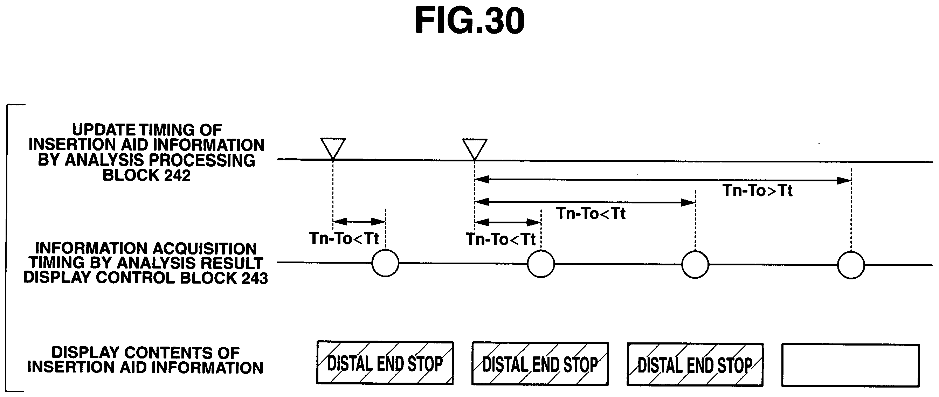

FIG. 30 shows the relationship among the timing when the analysis processing block 242 updates the insertion aid information, the timing when the analysis result display control block 243 acquires the insertion aid information 233c, and the display content of the character string information of the insertion aid information 233c displayed on the display 228 of the image processing apparatus 204 in the above-described working.

As shown in FIG. 30, the insertion aid information 233c is updated by the analysis processing block 242. The analysis result display control block 243 acquires the insertion aid information 233c at certain time intervals, and at that time, the analysis result display control block 243 performs the processing for determining whether or not the elapsed time satisfies the condition of Tn-T0<Tt. Then the analysis result display control block 243 maintains the display of the character string information of the insertion aid information 233c while the Tn-T0 is within the predetermined time Tt. Here, the display of "distal end stop" is maintained.

Therefore, even if the operator is late in observing the display surface of the display 228 of the image processing apparatus 204, the operator can certainly know (confirm) the insertion aid information 233c as long as the elapsed time is within the (threshold value Tt as) predetermined time.

Note that, though the above description uses the insertion shape data creation time T0 corresponding to the insertion aid information 233c as the time used for the determination whether or not to maintain the display, other time close to the insertion shape data creation time may be used. For example, the generation time or update time of the insertion aid information 233c may be used.

Thus, the display content of "distal end stop" is maintained, if the time elapsed from around the generation time of the insertion aid information 233c is within the predetermined time. However, after the time has elapsed over the predetermined time, the "distal end stop" is not displayed and display content is appropriately updated.

Thus, the present embodiment can effectively prevent or reduce the possibility for the operator to overlook the insertion aid information 233c, whereby the operator can securely confirm the insertion aid information 233c. Accordingly, operability with respect to the insertion operation at the time of endoscopy can be improved.

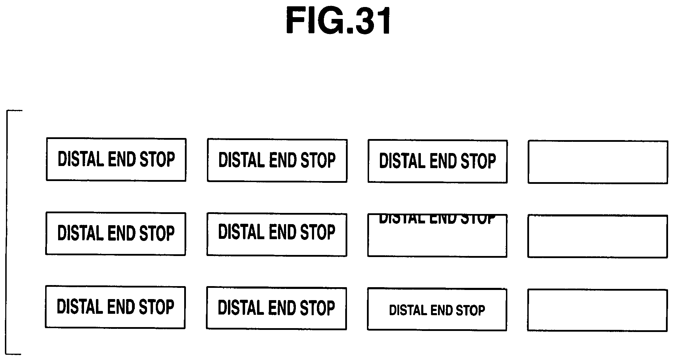

Note that, though in the present embodiment, the display/delete (non-display) of the insertion aid information 233c is controlled by the elapsed time from the time around the generation time of the insertion aid information 233c, the display color, display position, and display size may be changed according to the elapsed time as shown in FIG. 31.

That is, as shown by the diagonal lines, the "distal end stop" may be displayed in a display color such as red color within the predetermined elapsed time, and the display may be stopped (the display of "distal end stop" is deleted) when the elapsed time exceeds the predetermined elapsed time.

In addition, it may also be configured such that the display of "distal end stop" is performed within the predetermined elapsed time, and the display of "distal end stop" is moved by scrolling (from the display range thereof) from around the predetermined elapsed time, and the display is not performed when time has further elapsed, for example.

In addition, it may be configured such that the display of "distal end stop" is performed within the predetermined elapsed time, and the size of the character string showing the display of "distal end stop" is reduced from around the predetermined elapsed time, and the display is not performed when time has further elapsed, for example. Alternatively, these may be combined. Almost the same effects can be obtained also in such a case.

Moreover, though in the present embodiment, the analysis processing block 242 performs the analysis processing so as to generate from the insertion shape data the insertion aid information 233c corresponding to the response action state in which the distal end side is almost stopped with respect to the insertion operation of the insertion portion 211, the analysis processing block 242 may analyze from the insertion shape data the response action state of the insertion portion 211 with respect to the endoscope operation by the operator such as extraction operation, bending operation (angle operation), twisting operation of the electronic endoscope 206, and may perform determination processing for determining whether or not the operations are actually performed, to contain the determination result in the insertion aid information 233c.

For example, the response action state with respect to the extraction operation can be determined by determining the moving amount of the hand side of the insertion portion 211 and the moving amount of the distal end side of the insertion portion 211 and also the moving direction in the axial direction of the insertion portion 211.

In addition, the response action state with respect to the bending operation can be determined by performing a determination processing for determining whether or not the insertion shape is significantly changed at only the part closer to the distal end side than the part adjacent to the proximal end of the bending portion, for example.

In addition, though the display of the above-described insertion aid information is "distal end stop" and insertion aid is performed by showing the operator the situation in which the distal end portion 214 does not advance even if the hand side of the insertion portion 211 is further pushed, also in the case of other endoscope operations, the insertion aid information 233c corresponding to each of the endoscope operations may be shown.

In this case, it is only necessary to appropriately control the elapsed time in maintaining the display, depending on the content displayed as the insertion aid information.

As such, when determining the endoscope operation by the operator such as the extraction operation, bending operation, and twisting operation of the electronic endoscope 206, the analysis result display control block 243 stops at least the display of the character string information "distal end stop". Then, the analysis result display control block 243 displays the character string information of the insertion aid information 233c corresponding to the determined endoscope operation as needed. This makes it possible for the operator to confirm the state to check whether or not the response action of the insertion portion 211 is smoothly performed with respect to the extraction operation, angle operation, and twisting operation of the endoscope, thereby enabling the operability of the endoscope operation including the insertion operation to be improved.

(Fifth Embodiment)

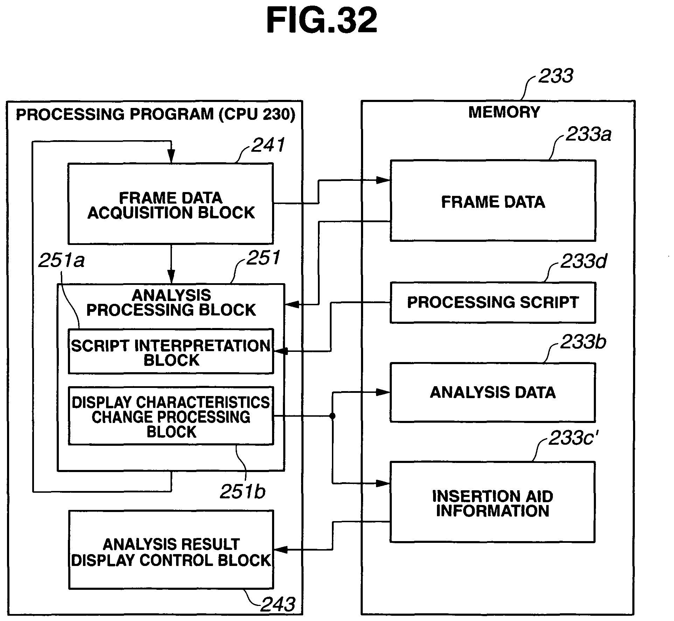

Next, the fifth embodiment of the present invention will be described with reference to FIGS. 32 to 34. The configuration of the in-vivo insertion monitoring system provided with the fifth embodiment is the same as that shown in FIG. 25. The present embodiment uses a processing program a part of which is different from the ' processing program stored in the program storage portion 232 in the image processing apparatus 204 in FIG. 25.

The function blocks realized like software using the processing program by the CPU 231 are shown in FIG. 32. The processing program shown in FIG. 32 is configured of the frame data acquisition block 241, an analysis processing block 251, and the analysis result display control block 243.

Here, the frame data acquisition block 241 and the analysis result display control block 243 perform the same processings as those shown in FIG. 28. On the other hand, the analysis processing block 251 in the present embodiment is configured of a script interpretation block 251a for interpreting a processing script 233d stored in the memory 233 and a display characteristics change processing block 251b for performing display characteristics change processing including the insertion aid information generation processing.

Thus, the analysis processing block 251, in addition to performing the analysis processing for generating insertion aid information 233c in the fourth embodiment, can generate insertion aid information 233c' corresponding to a plurality of kinds of condition by change setting of the condition setting contents, for example, and change the display characteristics so as to correspond to the insertion aid information 233c'.

Moreover, in the memory 233, the frame data 233a, the analysis data 233b, and insertion aid information 233c' are stored same as the case in FIG. 28. Furthermore, the present embodiment includes, as the processing program stored in the processing program storage portion 232, a processing script file describing the display characteristics processing procedures containing change of condition setting and the like, and the CPU 231 (See FIG. 25) reads out the processing script file at the time of activation, to store the read out processing script file on the memory 233 as the processing script 233d.

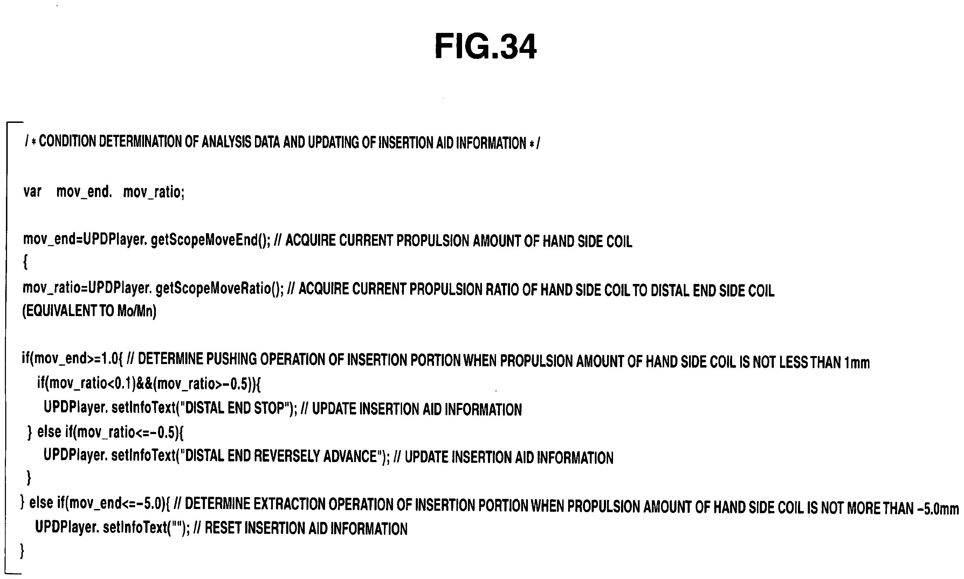

Note that the processing script 233d describes the processing contents including the generation processing of the insertion aid information in the analysis processing block 251 in conformity to, for example, the grammar of Java (registered trademark) Script as a predetermined programming language. The specific example of the processing script 233d is shown in FIG 34, for example. In the case of the fourth embodiment, the analysis processing block 42 determines, as shown in FIG. 28, whether or not the condition of M0/Mn<0.1 (corresponding to "distal end stop") is satisfied, and generates the insertion aid information to write the generated insertion aid information into the memory 233 when the condition is satisfied.

On the other hand, in the present embodiment, in the processing script 233d shown in FIG. 34, the processing contents (condition contents in the "if" part) with respect to the analysis data are changed so as to generate character string information of the insertion aid information 233c' showing "distal end reversely advance" in addition to that showing "distal end stop". In this case, in FIG. 34, extraction operation can be judged by checking the code (plus or minus) of the propulsion amount of the hand side coil.

In the fourth embodiment, the processing functions of the frame data acquisition block 241 and the like shown in FIG. 28 are executed by the CPU 231 at high speed in a language compiled and converted into an executable format from the programming language.

On the other hand, in the present embodiment, the script interpretation block 251a of the analysis processing block 251 sequentially interprets the contents of the programming language described in the processing script 233d into the language in executable format. Then, the display characteristics change processing block 251b sequentially performs the interpreted processings.

In this case, the display characteristics change processing block 251b executes processings such as acquirement of the analysis data 233b on the memory 233, condition determination, loop control, generation and updating of insertion aid information, and the like.

Thus, the analysis processing block 251 in the present embodiment consecutively interprets the analysis contents described in the processing script 233d to execute the analysis contents as an interpreter. Therefore, it is easier for the analysis processing block 251 to change the parameter values of the insertion aid information setting part, for example, during the action (inspection) without stopping the system, and execute the analysis contents at the changed values, respectively, to change to set the parameter values to the most appropriate values.

Thus, the processing contents such as display characteristics can be changed by changing description contents of the processing script 233d. For example, the display contents (included in the display characteristics) of the insertion aid information can be changed by changing the condition contents of the processing script as described above.

On the other hand, a file generated after compiling is executed in the fourth embodiment, therefore, even in a case of performing a small change, it is necessary to stop the system and change the contents of the processing program, to generate a file in executable format by compiling the processing program having the changed contents. In addition, the analysis processing cannot be performed unless the file is converted into executable format, so that it takes a lot of trouble with the work for just setting the parameter value to an appropriate value described above. Note that in the present embodiment, only the analysis processing block 251 is executed as the interpreter.

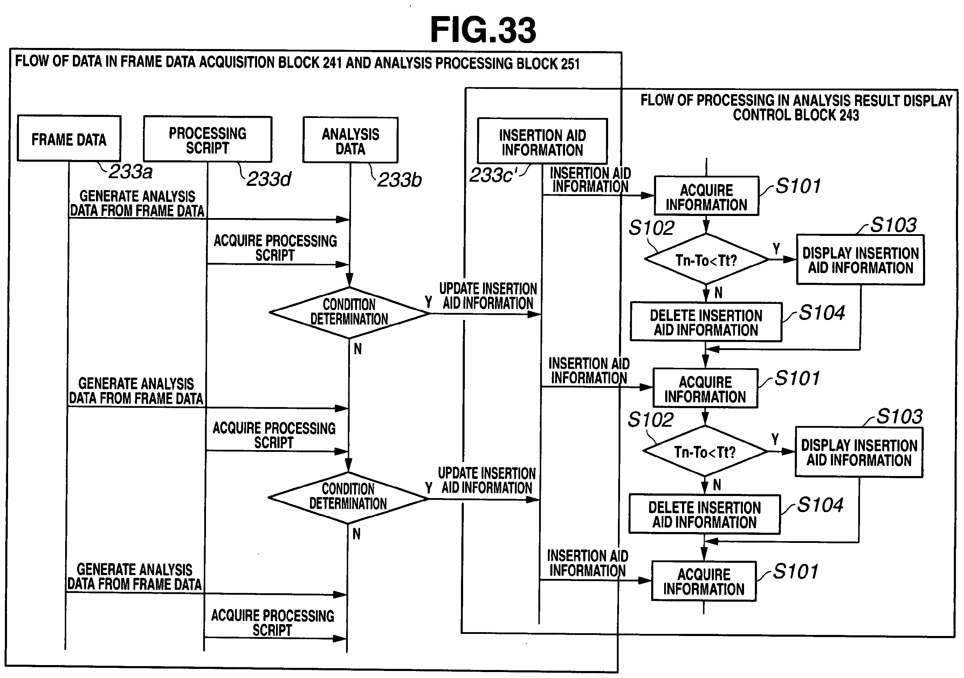

The action of the present embodiment with such a configuration will be described with reference to FIG. 33. Note that FIG. 33 shows a flow of data in the frame data acquisition block 241 and the analysis processing block 251 and a processing flow in the analysis result display control block 243.

The general outline of the flow of processing data in FIG. 33 is easily understood from the comparison with FIG. 29. Though the condition determination is performed to determine whether or not the condition of M0/Mn<0.1 (corresponding to "distal end stop") is satisfied in the analysis processing block 242 in FIG. 29, the condition determination is performed on the contents described by the processing script in FIG. 33. Hereinafter, more detailed description will be made.

The frame data acquisition block 241 records the frame data 233a on the memory 233.

Subsequently, the analysis processing block 251 performs analysis processing of the frame data 233a and thereafter acquires the processing script 233d from the memory 233. In the processing script 233d, the contents shown in FIG. 34 are described, for example. Next, the script interpretation block 251a interprets the processing script 233d. Then, based on the interpreted processing procedure, processings are performed by the display characteristics change processing block 251b.

As shown in FIG 33, (the display characteristics change processing block 251b in) the analysis processing block 251 acquires the interpreted analysis data 233b, and performs condition determination. When determining that the analysis data does not fall under the condition, the analysis processing block prepares for performing condition determination on the next frame data. On the other hand, when determining that the analysis data falls under the condition, the analysis processing block updates the insertion aid information 233c' generated as a result of determination of the analysis data falling under the condition, to write the updated information into the memory 233. Specifically, character string information "distal end stop" in the case of the fourth embodiment, and in addition, "distal end reversely advance" is written into the memory 233. Such processings are repeated.

On the other hand, the analysis result display control block 243, same as the case of the third embodiment, acquires the insertion aid information 233c' on the memory 233 at certain time intervals (step S101) and also acquires the current time Tn to determine whether or not the difference between the current time Tn and the insertion shape data creation time T0 of the insertion aid information 323c' is smaller than the threshold value Tt (Tn-T0<Tt) (step S102).

Then, if the condition of Tn-T0<Tt is satisfied, the analysis result display control block 243 displays the character string information of insertion aid information 233c' on the display 228 of the image processing apparatus 204 (step S103). In the present embodiment, the character string information of "distal end stop" and "distal end reversely advance" is displayed.

In addition, if the difference between the current time Tn and the insertion shape data creation time T0 of the insertion aid information 233c' is equal to or larger than a predetermined threshold value Tt (Tn-T0≥Tt), the character string information displayed on the display 228 of the image processing apparatus 204 is deleted (step S104).

Thus, the in-vivo insertion monitoring system 201 is activated according to the contents described in the processing script 233d, so that it is unnecessary to newly create a processing program, whereby facilitating a detailed customization of action contents and the like.

Note that in the in-vivo insertion monitoring system 201, a function for reading the processing script at an arbitrary timing may be added, thereby allowing the amended or selected processing script to be read with respect to operator's instruction without terminating the action of the in-vivo insertion monitoring system 201.

Accordingly, the present embodiment has effects described below.

Detailed customization can be easily realized without newly recreating a processing program of the in-vivo insertion monitoring system.

Furthermore, the customization can be performed at the time of setting adjustment and inspection without stopping the in-vivo insertion monitoring system, thereby facilitating instant confirmation of the customization result and enabling the insertion aid information to be continuously (smoothly) presented while amending/selecting the generation method of the insertion aid information during the inspection. In addition, it is possible to appropriately display various kinds of insertion aid information by which the operability for an operator can be improved. In addition to the above, the present embodiment has the same effects as those in the fourth embodiment.

Note that, by changing the contents of the processing script shown in FIG. 34, it is possible to analyze the response action state of the insertion portion 211 with respect to the endoscope operation such as bending operation and display the analysis result as the insertion aid information in a case of a predetermined response action state.

Note that, in the case of displaying the insertion aid information, the device on which the information is displayed is not limited to the display 228 of the image processing apparatus 204. The insertion aid information may be displayed, for example, on the display 222 of the endoscope insertion shape observation apparatus 203 or on the observation monitor 209 of the endoscope apparatus 202, or the display device on which the insertion aid information is displayed may be selected and set. In addition, in the case of displaying insertion aid information, no limitation is placed on the display by character string information. The insertion aid information of "distal end stop", for example, may be notified to the operator by changing the display color of the background portion of the insertion shape displayed on the display 222 of the endoscope insertion shape observation apparatus 203, for example.

Note that embodiments and the like configured by partly combining each of the above-described embodiments also belong to the present invention. The present invention is not limited to the above-described embodiments, various changes and modifications are possible without changing the scope of the present invention.

**Claims**

1. An endoscope insertion shape analysis apparatus (1) comprising:

    an endoscope (21) including an insertion portion (21a) to be inserted into a body cavity;
    shape detection means (31, 32) for detecting a shape of the insertion portion (21a) of the endoscope (21);
    shape analysis means (41) for determining by analysis a specific position or a specific portion from the shape detected by the shape detection means (31, 32), based on information detected by the shape detection means (31, 32); and
    pattern classification means (41) for classifying a static shape of the insertion portion (21a) of the endoscope (21) into a pattern among patterns of a linear shape, a walking stick shape, a middle folding shape, a loop shape, and a flexure shape, based on analysis result obtained by analysis by the shape analysis means (41), the pattern classification means (41) are configured to acquire static pattern codes obtained by coding the static shape based on the analysis result and store the acquired static pattern codes in a ring buffer, wherein
    the pattern classification means (41) are configured to acquire N pieces of the static pattern codes stored in the buffer, generate a dynamic pattern code integrating the sequentially acquired N pieces of static pattern codes and the static pattern code acquired last based on the analysis result, and classify a dynamic shape of the insertion portion (21a) of the endoscope (21) into a pattern based on the dynamic pattern code.

2. The endoscope insertion shape analysis apparatus (1) according to Claim 1, wherein the plurality of static pattern codes generating the dynamic pattern code are static pattern codes based on analysis results continuous in time obtained by analysis by the shape analysis means (41).

3. The endoscope insertion shape analysis apparatus (1) according to Claim 1 or 2, further comprising,

information storage means (41f) for storing a dynamic dictionary file in which the dynamic pattern code and predetermined pattern information corresponding to the dynamic pattern code are associated with each other, wherein the pattern classification means (41) classifies the dynamic shape into a pattern based on the dynamic dictionary file.

4. The endoscope insertion shape analysis apparatus (1) according to Claim 3, wherein the pattern information includes operation aid information for inserting the endoscope (21) into the body cavity.

5. The endoscope insertion shape analysis apparatus (1) according to Claim 3 or 4, wherein the information storage means (41f) stores a correction code file for correcting the dynamic pattern code, and the pattern classification means (41) corrects the dynamic pattern code based on the correction code file, and classifies the dynamic shape into a pattern based on the dynamic dictionary file.

6. The endoscope insertion shape analysis apparatus (1) according to Claim 1 further comprising, information storage means (41f) for storing static dictionary file in which the static pattern code and predetermined pattern information corresponding to the static pattern code are associated with each other, wherein the pattern classification means (41) classifies the static shape into a pattern based on the static dictionary file.

7. The endoscope insertion shape analysis apparatus (1) according to Claim 6, wherein the pattern information includes operation aid information for inserting the endoscope (21) into the body cavity.

8. The endoscope insertion shape analysis apparatus (1) according to Claim 6 or 7, wherein the information storage means (41f) stores a correction code file to correct the static pattern code, and the pattern classification means (41) corrects the static pattern code based on the correction code file and classifies the static shape into a pattern based on the static dictionary file.

9. The endoscope insertion shape analysis apparatus (1) according to any one of Claims 1 to 8, further comprising:

data output destination registration means for registering an output destination of shape data of a shape of the insertion portion of the endoscope (21) detected by the shape detection means; and data output means for outputting the shape data to the output destination registered by the data output destination registration means.

10. The endoscope insertion shape analysis apparatus (1) according to any one of Claims 1 to 9, further comprising display means (44) for displaying an endoscope image picked up by the endoscope (21) and the shape detected by the shape detection means (31, 32).

**Patentansprüche**

1. Vorrichtung zur Analyse einer Endoskop-Einführungsform (1), umfassend:

ein Endoskop (21), das einen Einführungsabschnitt (21a) umfasst, der in eine Körperhöhle einzuführen ist; ein Formerkennungsmittel (31, 32) zum Erkennen einer Form des Einführungsabschnitts (21a) des Endoskops (21); ein Formanalysemittel (41) zum Bestimmen einer spezifischen Position oder eines spezifischen Abschnitts der von dem Formerkennungsmittel (31, 32) erkannten Form durch Analyse, basierend auf von dem Formerkennungsmittel (31, 32) erkannter Information; und Musterklassifizierungsmittel (41) zum Klassifizieren einer statischen Form des Einführungsabschnitts (21a) des Endoskops (21) in ein Muster aus Mustern einer linearen Form, einer Gehstockform, einer in der Mitte gefalteten Form, einer Schlaufenform und einer Form mit Beugung basierend auf einem Analyseergebnis, das durch Analyse durch das Formanalysemittel (41) erhalten wurde, wobei das Musterklassifizierungsmittel (41) dazu ausgelegt ist, statische Mustercodes zu erfassen, die durch Kodieren der statischen Form basierend auf dem Analyseergebnis erhalten wurden, und die erfassten statischen Mustercodes in einem Ringpuffer zu speichern, wobei das Musterklassifizierungsmittel (41) dazu ausgelegt ist, N-Stücke der in dem Puffer gespeicherten statischen Mustercodes zu erfassen, einen dynamischen Mustercode zu erzeugen, der die sequenziell erfassten N-Stücke der statischen Mustercodes und den statischen Mustercode integriert, der zuletzt basierend auf dem Analyseergebnis erfasst wurde, und eine dynamische Form des Einführungsabschnitts (21a) des Endoskops (21) basierend auf dem dynamischen Mustercode in ein Muster zu klassifizieren.

2. Vorrichtung zur Analyse einer Endoskop-Einführungsform (1) nach Anspruch 1, wobei die Vielzahl von statischen Mustercodes, die den dynamischen Mustercode erzeugen, statische Mustercodes basierend auf Analyseergebnissen sind, die im kontinuierlichen Zeitverlauf durch Analyse durch das Formanalysemittel (41) erhalten wurden.

3. Vorrichtung zur Analyse einer Endoskop-Einführungsform (1) nach Anspruch 1 oder 2, ferner umfassend
ein Informationsspeichermittel (41f) zum Speichern einer dynamischen Verzeichnisdatei, in der der dynamische Mustercode und die dem dynamischen Mustercode entsprechende vorbestimmte Musterinformation miteinander verknüpft sind,
wobei das Musterklassifizierungsmittel (41) die dynamische Form basierend auf der dynamischen Verzeichnisdatei in ein Muster klassifiziert.

4. Vorrichtung zur Analyse einer Endoskop-Einführungsform (1) nach Anspruch 3, wobei die Musterinformation Bedienungshilfsinformation zur Einführung des Endoskops (21) in die Körperhöhle umfasst.

5. Vorrichtung zur Analyse einer Endoskop-Einführungsform (1) nach Anspruch 3 oder 4, wobei das Informationsspeichermittel (41f) eine Korrekturcodedatei zum Korrigieren des dynamischen Mustercodes speichert und das Musterklassifizierungsmittel (41) den dynamischen Mustercode basierend auf der Korrekturcodedatei korrigiert und die dynamische Form in ein Muster basierend auf der dynamischen Verzeichnisdatei klassifiziert.

6. Vorrichtung zur Analyse einer Endoskop-Einführungsform (1) nach Anspruch 1, ferner umfassend
ein Informationsspeichermittel (41f) zum Speichern einer statischen Verzeichnisdatei, in der der dynamische Mustercode und die dem dynamischen Mustercode entsprechende vorbestimmte Musterinformation miteinander verknüpft sind,
wobei das Musterklassifizierungsmittel (41) die statische Form basierend auf der statischen Verzeichnisdatei in ein Muster klassifiziert.

7. Vorrichtung zur Analyse einer Endoskop-Einführungsform (1) nach Anspruch 6, wobei die Musterinformation Bedienungshilfsinformation zur Einführung des Endoskops (21) in die Körperhöhle umfasst.

8. Vorrichtung zur Analyse einer Endoskop-Einführungsform (1) nach Anspruch 6 oder 7, wobei das Informationsspeichermittel (41f) eine Korrekturcodedatei speichert, um den statischen Mustercode zu korrigieren und das Musterklassifizierungsmittel (41) den statischen Mustercode basierend auf der Korrekturcodedatei korrigiert und die statische Form basierend auf der statischen Verzeichnisdatei in ein Muster klassifiziert.

9. Vorrichtung zur Analyse einer Endoskop-Einführungsform (1) nach einem der Ansprüche 1 bis 8, ferner umfassend:

ein Datenausgabezielregistrierungsmittel zum Registrieren eines Ausgabeziels von Formdaten einer Form des Einführungsabschnitts des Endoskops (21), die von dem Formerkennungsmittel erkannt werden; und
ein Datenausgabemittel zum Ausgeben der Formdaten an das Ausgabeziel, das von dem Datenausgabezielregistrierungsmittel registriert wurde.

10. Vorrichtung zur Analyse einer Endoskop-Einführungsform (1) nach einem der Ansprüche 1 bis 9, ferner umfassend ein Anzeigemittel (44) zum Anzeigen eines Endoskopbilds, das von dem Endoskop (21) aufgenommen wurde, und der Form, die von dem Formerkennungsmittel (31, 32) erkannt wurde.

## Revendications

1. Appareil d'analyse de forme d'insertion d'endoscope (1) comprenant :

un endoscope (21) comprenant une partie d'insertion (21a) destinée à être insérée dans une cavité corporelle ;
des moyens de détection de forme (31, 32) destinés à détecter une forme de la partie d'insertion (21a) de l'endoscope (21) ;
des moyens d'analyse de forme (41) destinés à déterminer par analyse une position spécifique ou une partie spécifique à partir de la forme détectée par les moyens de détection de forme (31, 32), sur la base d'informations détectées par les moyens de détection de forme (31, 32) ; et
des moyens de classification de motif (41) destinés à classifier une forme statique de la partie d'insertion (21a) de l'endoscope (21) en un motif parmi des motifs d'une forme linéaire, d'une forme de canne de marche, d'une forme à pliage intermédiaire, d'une forme de boucle et d'une forme fléchie, sur la base d'un résultat d'analyse obtenu par analyse par les moyens d'analyse de forme (41), les moyens de classification de motif (41) étant configurés pour acquérir des codes de motif statiques obtenus par codage de la forme statique sur la base du résultat d'analyse et stocker les codes de motif statiques acquis dans une mémoire tampon circulaire,

les moyens de classification de motif (41) étant configurés pour acquérir N éléments des codes de motif statiques stockés dans la mémoire tampon, générer un code de motif dynamique intégrant les N éléments acquis séquentiellement de codes de motif statiques et le code de motif statique acquis en dernier sur la base du résultat d'analyse, et classifier une forme dynamique de la partie d'insertion (21a) de l'endoscope (21) en un motif sur la base du code de motif dynamique.

2. Appareil d'analyse de forme d'insertion d'endoscope (1) selon la revendication 1, dans lequel la pluralité de codes de motif statiques générant le code de motif dynamique sont des codes de motif statiques basés sur des résultats d'analyse continus dans le temps obtenus par analyse par les moyens d'analyse de forme (41).

3. Appareil d'analyse de forme d'insertion d'endoscope (1) selon la revendication 1 ou 2, comprenant en outre,

des moyens de stockage d'informations (41f) destinés à stocker un fichier de dictionnaire dynamique dans lequel le code de motif dynamique et des informations de motif prédéterminées correspondant au code de motif dynamique sont associés les uns aux autres,

les moyens de classification de motif (41) classifiant la forme dynamique en un motif sur la base du fichier de dictionnaire dynamique.

4. Appareil d'analyse de forme d'insertion d'endoscope (1) selon la revendication 3, dans lequel les informations de motif comprennent des informations d'aide à l'opération pour l'insertion de l'endoscope (21) dans la cavité corporelle.

5. Appareil d'analyse de forme d'insertion d'endoscope (1) selon la revendication 3 ou 4, dans lequel les moyens de stockage d'informations (41f) stockent un fichier de code de correction pour la correction du code de motif dynamique, et les moyens de classification de motif (41) corrigent le code de motif dynamique sur la base du fichier de code de correction, et classifient la forme dynamique en un motif sur la base du fichier de dictionnaire dynamique.

6. Appareil d'analyse de forme d'insertion d'endoscope (1) selon la revendication 1, comprenant en outre, des moyens de stockage d'informations (41f) destinés à stocker un fichier de dictionnaire statique dans lequel le code de motif statique et des informations de motif prédéterminées correspondant au code de motif statique sont associés les uns aux autres, les moyens de classification de motif (41) classifiant la forme statique en un motif sur la base du fichier de dictionnaire statique.

7. Appareil d'analyse de forme d'insertion d'endoscope (1) selon la revendication 6, dans lequel les informations de motif comprennent des informations d'aide à l'opération pour l'insertion de l'endoscope (21) dans la cavité corporelle.

8. Appareil d'analyse de forme d'insertion d'endoscope (1) selon la revendication 6 ou 7, dans lequel les moyens de stockage d'informations (41f) stockent un fichier de code de correction pour corriger le code de motif statique, et les moyens de classification de motif (41) corrigent le code de motif statique sur la base du fichier de code de correction et classifient la forme statique en un motif sur la base du fichier de dictionnaire statique.

9. Appareil d'analyse de forme d'insertion d'endoscope (1) selon l'une quelconque des revendications 1 à 8, comprenant en outre :

des moyens d'enregistrement de destination de sortie de données destinés à enregistrer une destination de sortie de données de forme d'une forme de la partie d'insertion de l'endoscope (21) détectée par les moyens de détection de forme ; et

des moyens de sortie de données destinés à délivrer les données de forme à la destination de sortie enregistrée par les moyens d'enregistrement de destination de sortie de données.

10. Appareil d'analyse de forme d'insertion d'endoscope (1) selon l'une quelconque des revendications 1 à 9, comprenant en outre des moyens d'affichage (44) destinés à afficher une image d'endoscope capturée par l'endoscope (21) et la forme détectée par les moyens de détection de forme (31, 32).

# FIG.1

# FIG.2

COORDINATES OF COIL 0 IN
j-TH FRAME $(X^j_0, Y^j_0, Z^j_0)$

DISTAL END
SIDE OF
INSERTION
PORTION

COORDINATES OF COIL 1 IN
j-TH FRAME $(X^j_1, Y^j_1, Z^j_1)$

Y

Z

VISUAL
POINT

VISUAL AXIS
VECTOR

PROXIMAL END SIDE OF
INSERTION PORTION
(HAND SIDE)

COORDINATES OF COIL M-1 IN
j-TH FRAME $(X^j_{M-1}, Y^j_{M-1}, Z^j_{M-1})$

X

# FIG.3

| INSERTION SHAPE DATA | ZEROTH FRAME PACKET | FIRST FRAME PACKET | ... | j-TH FRAME PACKET | ... | n-1TH FRAME PACKET |
|---|---|---|---|---|---|---|

| FRAME PACKET | TYPE | INSERTION SHAPE DATA CREATION TIME | DISPLAY ATTRIBUTE | ATTACHED INFORMATION | COORDINATES OF COILS |
|---|---|---|---|---|---|

| COORDINATES OF COILS | $(X^j_0, Y^j_0, Z^j_0)$ | $(X^j_1, Y^j_1, Z^j_1)$ | | $(X^j_{M-1}, Y^j_{M-1}, Z^j_{M-1})$ |
|---|---|---|---|---|

DISTAL END OF
INSERTION PORTION ⇐

ROOT OF
⇒ INSERTION PORTION

# FIG.4

EP 1 917 901 B1

# FIG.5

```
                    ( START )
                        │
                        ▼                              S1
    ┌───────────────────────────────────────────────────┐
    │          LOAD INITIALIZATION INFORMATION           │
    └───────────────────────────────────────────────────┘
                        │
                        ▼                              S2
    ┌───────────────────────────────────────────────────┐
    │        SET MODE FOR RECEIVING AND SAVING DATA      │
    └───────────────────────────────────────────────────┘
                        │ ◄─────────────────────────────────┐
                        ▼          S3                        │
            ◇─────────────────────────────────◇  NO         │
            ◇       INSPECTION STARTED?        ◇ ────────────┘
                        │ YES                  S4
    ┌───────────────────────────────────────────────────┐
    │       START COMMUNICATION WITH INSERTION SHAPE     │
    │                OBSERVATION APPARATUS               │
    └───────────────────────────────────────────────────┘
                        │                              S5
    ┌───────────────────────────────────────────────────┐
    │       START COMMUNICATION WITH VIDEO PROCESSOR     │
    └───────────────────────────────────────────────────┘
                        │                              S6
    ┌───────────────────────────────────────────────────┐
    │    TRANSMIT INSPECTION INFORMATION ACQUISITION COMMAND  │
    └───────────────────────────────────────────────────┘
                        │                              S7
    ┌───────────────────────────────────────────────────┐
    │        SAVE INSPECTION INFORMATION IN DATABASE     │
    └───────────────────────────────────────────────────┘
                        │ ◄─────────────────────────────────┐
                        ▼                              S8    │
    ┌───────────────────────────────────────────────────┐   │
    │          TRANSMIT SHAPE ACQUISITION COMMAND        │   │
    └───────────────────────────────────────────────────┘   │
                        │                              S9    │
    ┌───────────────────────────────────────────────────┐   │
    │  SAVE SHAPE DATA IN ASSOCIATION WITH INSPECTION INFORMATION │
    └───────────────────────────────────────────────────┘   │
                        │                              S10   │
    ┌───────────────────────────────────────────────────┐   │
    │   COMPRESS MOVING IMAGE TO SAVE COMPRESSED IMAGE   │   │
    │       IN ASSOCIATION WITH INSPECTION INFORMATION   │   │
    └───────────────────────────────────────────────────┘   │
                        │                              S11   │
    ┌───────────────────────────────────────────────────┐   │
    │             SHAPE ANALYSIS PROCESSING              │   │
    └───────────────────────────────────────────────────┘   │
                        │                    S12             │
            ◇─────────────────────────────────◇  NO         │
            ◇      INSPECTION TERMINATED?      ◇ ────────────┘
                        │ YES                  S13
    ┌───────────────────────────────────────────────────┐
    │       COMMUNICATION TERMINATION PROCESSING         │
    └───────────────────────────────────────────────────┘
                        │
                        ▼
                    ( END )
```

# FIG.6

```
( SHAPE ANALYSIS PROCESSING )
                │
                ▼                                    ╱S21
┌──────────────────────────────────────────────────────┐
│    ACQUIRE INSERTION SHAPE DATA OF PREVIOUS FRAME      │
└──────────────────────────────────────────────────────┘
                │                                    ╱S22
                ▼
┌──────────────────────────────────────────────────────┐
│     EXECUTE VARIOUS KINDS OF ANALYSIS PROCESSINGS      │
└──────────────────────────────────────────────────────┘
                │                                    ╱S23
                ▼
┌──────────────────────────────────────────────────────┐
│          CODE ANALYSIS PROCESSING RESULT              │
└──────────────────────────────────────────────────────┘
                │                                    ╱S24
                ▼
┌──────────────────────────────────────────────────────┐
│      RETRIEVE DICTIONARY USING CODE AS KEY AND         │
│           DISPLAY ACQUISITION RESULT                  │
└──────────────────────────────────────────────────────┘
                │                                    ╱S25
                ▼
┌──────────────────────────────────────────────────────┐
│      GENERATE AND DISPLAY TWO-DIMENSIONALLY           │
│      PROJECTED ENDOSCOPE SHAPE IMAGE                   │
└──────────────────────────────────────────────────────┘
                │
                ▼
          ( RETURN )
```

# FIG.7

| DETERMINATION OF INSERTION LENGTH | | |
|---|---|---|
| 0~100: | 00 | |
| 100~200: | 01 | |
| 200~300: | 10 | |
| 300~ | : 11 | |

DETERMINATION OF LOOP/HELIX
NO: 00
RIGHT ROTATION: 01
LEFT ROTATION: 10

ANGLE WITH RESPECT TO ROOT OF BENDING PORTION
0~90°: 0
90°~ : 1

ANGLE WITH RESPECT TO ROOT OF DISTAL END PORTION
0~90°: 0
90°~ : 1

BENDING OF NOT MORE THAN 60mm OF RADIUS OF CURVATURE AT INSERTION PORTION
YES: 0
NO: 1

DETERMINATION OF ANGLE
0~90°: 0
90°~ : 1

```
┌───┬───┬───┬───┬───┬───┬───┬───┐
│ 0 │ 0 │ 1 │ 0 │ 1 │ 1 │ 0 │ 1 │
└───┴───┴───┴───┴───┴───┴───┴───┘
```

CODE REGARDING AS STRING OF EIGHT BITS (1 BYTE)

CODE=0x2D

# FIG.8

DICTIONARY

| CODE | ENDOSCOPE SHAPE ID | ENDOSCOPE SHAPE CLASSIFICATION INFORMATION | ENDOSCOPE SHAPE CLASSIFICATION AID INFORMATION | OPERATION AID INFORMATION |
|------|------|------|------|------|
| 0x00 | 1 | LINEAR | – | – |
| 0x01 | 2 | WALKING STICK | – | INSERTION OPERATION WHILE REDUCING ANGLE |
| 0x02 | 3 | MIDDLE FOLDING | – | – |
| 0x03 | 4 | FLEXURE | – | – |
| 0x04 | 5 | LOOP | RIGHT ROTATION | RELEASE DIRECTION: RIGHT ROTATION |
| 0x05 | 4 | FLEXURE | RIGHT ROTATION | RELEASE DIRECTION: RIGHT ROTATION |
| ⋮ | ⋮ | ⋮ | ⋮ | ⋮ |

# FIG.9

**LINEAR SHAPE**

# FIG.10

**WALKING STICK SHAPE**

# FIG.11

**MIDDLE FOLDING SHAPE**

# FIG.12

**LOOP SHAPE**

# FIG.13

**FLEXURE SHAPE**

EP 1 917 901 B1

# FIG.14

(RING) BUFFER

CURRENT BUFFER POSITION: REMAINDER OF COUNT/8

PAST N-TH BUFFER POSITION: REMAINDER OF (COUNT-N)/8

# FIG.15

( SHAPE ANALYSIS PROCESSING )

S21
ACQUIRE INSERTION SHAPE DATA OF PREVIOUS FRAME

S22
EXECUTE VARIOUS KINDS OF ANALYSIS PROCESSINGS

S23
CODE ANALYSIS PROCESSING RESULT

S31
ACQUIRE PAST N-NUMBER OF CODES

S24
RETRIEVE DICTIONARY USING CODE SEQUENCE AS KEY AND DISPLAY ACQUISITION RESULT

S32
STORE CODE IN BUFFER

S25
GENERATE AND DISPLAY TWO-DIMENSIONALLY PROJECTED ENDOSCOPE SHAPE IMAGE

( RETURN )

29

# FIG.16

DETERMINATION
OF INSERTION LENGTH

0~100:     00
100~200:  01
200~300:  10
300~      :  11

ANGLE WITH RESPECT
TO ROOT OF BENDING
PORTION

0~90°:   0
90°~   :  1

BENDING OF NOT MORE THAN
60mm OF RADIUS OF CURVATURE
AT INSERTION PORTION

YES:  0
NO:   1

DETERMINATION OF
LOOP/HELIX

NO:                      00
RIGHT ROTATION: 01
LEFT ROTATION:   10

ANGLE WITH RESPECT
TO ROOT OF DISTAL
END PORTION

0~90°:   0
90°~   :  1

DETERMINATION
OF ANGLE

0~90°:   0
90°~   :  1

| 0 | 0 | 1 | 0 | 1 | 1 | 0 | 1 |
|---|---|---|---|---|---|---|---|

CODE REGARDING AS STRING
OF EIGHT BITS (1 BYTE)

CODE=0x2D

ACQUIRE CODES OF
PAST THREE TIMES IN BUFFER

| CODE=0x1D | CODE=0x1D | CODE=0x1D |
|---|---|---|

RING
BUFFER

AFTER DICTIONARY RETRIEVAL/ACQUISITION
BY FOUR-BYTE CODE, STORE THE ACQUIRED
CODE IN BUFFER

CODE=0x2D

# FIG.17

DICTIONARY

| CODE | ENDOSCOPE SHAPE ID | ENDOSCOPE SHAPE CLASSIFICATION INFORMATION | ENDOSCOPE SHAPE CLASSIFICATION AID INFORMATION | OPERATION AID INFORMATION |
|---|---|---|---|---|
| 0x00000000 | 1 | LINEAR | – | – |
| 0x00000001 | 2 | WALKING STICK | – | INSERTION OPERATION WHILE REDUCING ANGLE |
| 0x00000002 | 3 | MIDDLE FOLDING | – | – |
| 0x00000003 | 4 | FLEXURE | – | – |
| 0x00000004 | 5 | LOOP | RIGHT ROTATION | RELEASE DIRECTION: RIGHT ROTATION |
| 0x00000005 | 4 | FLEXURE | RIGHT ROTATION | RELEASE DIRECTION: RIGHT ROTATION |
| ⋮ | ⋮ | ⋮ | ⋮ | ⋮ |

# FIG.18

CORRECTION DICTIONARY

| CODE | CORRECTION CODE |
|---|---|
| 0x00000000 | 0x00000000 |
| 0x00000001 | 0x00000001 |
| 0x00000002 | 0x00000002 |
| 0x00000003 | 0x00000003 |
| ⋮ | ⋮ |
| 0x1D1D1D2D | 0x1D1D1D1D |
| 0x1D1D1D2E | 0x1D1D1D1E |
| ⋮ | ⋮ |

31

# FIG.19

# FIG.20

EP 1 917 901 B1

# FIG.21

| SPECIFY OUTPUT DESTINATION OF INSERTION SHAPE DATA |
|---|

OUTPUT DESTINATION *171*

☑ IMAGE FILING APPARATUS (THIRD COMMUNICATION PORT)

☐ INSERTION SHAPE DATA RECORDING APPLICATION

☑ INSERTION SHAPE DISPLAY APPLICATION

☐ INSERTION SHAPE ANALYSIS APPLICATION

*172*

| OK | CANCEL |
|---|---|

# FIG.22

**INSERTION SHAPE DATA MANAGEMENT APPLICATION SECOND THREAD**

**IMAGE FILING APPARATUS**

WAIT TRANSMISSION REQUEST

ACQUIRE INSERTION SHAPE DATA FROM BUFFER IN SHARED MEMORY

REQUEST TRANSMISSION OF INSERTION SHAPE DATA THROUGH THIRD COMMUNICATION PORT

TRANSMIT INSERTION SHAPE DATA THROUGH THIRD COMMUNICATION PORT

COMPLETE TRANSMISSION OF INSERTION SHAPE DATA THROUGH THIRD COMMUNICATION PORT

# FIG.23

ENDOSCOPE INSERTION
SHAPE OBSERVATION
APPARATUS

INSERTION SHAPE
DATA MANAGEMENT
APPLICATION

FIRST THREAD

INSERTION SHAPE
ANALYSIS APPLICATION

REQUEST TRANSMISSION
OF INSERTION SHAPE
DATA THROUGH SECOND
COMMUNICATION PORT

TRANSMIT INSERTION
SHAPE DATA THROUGH
SECOND COMMUNICATION
PORT

COMPLETE TRANSMISSION
OF INSERTION SHAPE
DATA THROUGH THIRD
COMMUNICATION PORT

STORE IN BUFFER IN
SHARED MEMORY

TRANSMIT MESSAGE
TO INSERTION SHAPE
ANALYSIS APPLICATION

EXTRACT INSERTION SHAPE
DATA BASED ON ARGUMENT
OF MESSAGE

CONFIRM PROCESSING
COMPLETION OF
INSERTION SHAPE
ANALYSIS APPLICATION

COMPLETE INSERTION
SHAPE DATA EXTRACTION

EP 1 917 901 B1

# FIG.24

START

LOAD INITIALIZATION INFORMATION ╱S1

SET MODE FOR RECEIVING AND SAVING DATA ╱S2

INSPECTION STARTED? ╱S3 — NO

YES

START COMMUNICATION WITH VIDEO PROCESSOR ╱S5

TRANSMIT INSPECTION INFORMATION ACQUISITION COMMAND ╱S6

SAVE INSPECTION INFORMATION IN DATABASE ╱S7

ACQUIRE SHAPE DATA WHEN RECEIVING MESSAGE ╱S41

SAVE SHAPE DATA IN ASSOCIATION WITH INSPECTION INFORMATION ╱S9

COMPRESS MOVING IMAGE TO SAVE COMPRESSED IMAGE IN ASSOCIATION WITH INSPECTION INFORMATION ╱S10

SHAPE ANALYSIS PROCESSING ╱S11

INSPECTION TERMINATED? ╱S12 — NO

YES

COMMUNICATION TERMINATION PROCESSING ╱S13

END

# FIG.25

**ENDOSCOPE APPARATUS**

215
214 { 216 209
$C_0$
206
$C_1$
211
$C_{M-1}$

OBSERVATION MONITOR

IMAGE PICKUP SIGNAL
208
RGB SIGNAL

VIDEO PROCESSOR

207
208a
212
213
SYNCHRONIZATION

202

LIGHT SOURCE DEVICE

**ENDOSCOPE INSERTION SHAPE OBSERVATION APPARATUS**

222
DISPLAY

DRIVING SIGNAL
RGB SIGNAL

219 221
SENSE COIL UNIT → SHAPE PROCESSING DEVICE

203
221a

201
204

**IMAGE PROCESSING APPARATUS**

226
MOUSE

227
KEYBOARD

225a PC
MOVING IMAGE INPUT BOARD

Y/C
233

INSPECTION INFORMATION
225b
COMMUNICATION PORT

MEMORY

INSERTION SHAPE DATA
225c 231
COMMUNICATION PORT

CPU

PROCESSING PROGRAM STORAGE PORTION

HDD

232 234
225

RGB SIGNAL
228
DISPLAY

# FIG.26

**COORDINATES OF SOURCE COIL $C_0$ IN j-1TH FRAME**
$(X^j_0, Y^j_0, Z^j_0)$

**DISTAL END SIDE**

**COORDINATES OF SOURCE COIL $C_1$ IN j-1TH FRAME**
$(X^j_1, Y^j_1, Z^j_1)$

**COORDINATES OF SOURCE COIL $C_{M-1}$ IN j-1TH FRAME**
$(X^j_{M-1}, Y^j_{M-1}, Z^j_{M-1})$

**HAND SIDE**

# FIG.27

**INSERTION SHAPE DATA**

| ZEROTH FRAME DATA | FIRST FRAME DATA | ... | i-TH FRAME DATA | ... | n-1TH FRAME DATA |
|---|---|---|---|---|---|

**FRAME DATA**

| INSERTION SHAPE DATA CREATION TIME | DISPLAY ATTRIBUTE | ATTACHED INFORMATION | COORDINATES OF COILS |
|---|---|---|---|

**COORDINATES OF COILS**

| $(X^j_0, Y^j_0, Z^j_0)$ | $(X^j_1, Y^j_1, Z^j_1)$ | ... | $(X^j_{M-1}, Y^j_{M-1}, Z^j_{M-1})$ |
|---|---|---|---|
| DISTAL END OF INSERTION PORTION | | | PROXIMAL END OF INSERTION PORTION |

# FIG.28

*233*

**PROCESSING PROGRAM (CPU 231)**

**MEMORY**

*241*

FRAME DATA ACQUISITION BLOCK

*242*

ANALYSIS PROCESSING BLOCK

*243*

ANALYSIS RESULT DISPLAY CONTROL BLOCK (INSERTION AID INFORMATION DISPLAY CONTROL BLOCK)

*233a*

FRAME DATA

*233b*

ANALYSIS DATA

*233c*

INSERTION AID INFORMATION

# FIG.29

FLOW OF DATA IN FRAME DATA ACQUISITION BLOCK 241 AND ANALYSIS PROCESSING BLOCK 242

FRAME DATA ⟍233a

ANALYSIS DATA ⟍233b

INSERTION AID INFORMATION 233c

FLOW OF PROCESSING IN ANALYSIS RESULT DISPLAY CONTROL BLOCK 243

GENERATE ANALYSIS DATA FROM FRAME DATA

Mo/Mn<0.1?

UPDATE INSERTION AID INFORMATION

INSERTION AID INFORMATION

ACQUIRE INFORMATION — S101

S102 — Tn-To<Tt? — Y — DISPLAY INSERTION AID INFORMATION S103

N

DELETE INSERTION AID INFORMATION — S104

GENERATE ANALYSIS DATA FROM FRAME DATA

Mo/Mn<0.1?

UPDATE INSERTION AID INFORMATION

INSERTION AID INFORMATION

ACQUIRE INFORMATION — S101

S102 — Tn-To<Tt? — Y — DISPLAY INSERTION AID INFORMATION S103

N

DELETE INSERTION AID INFORMATION — S104

GENERATE ANALYSIS DATA FROM FRAME DATA

Mo/Mn<0.1?

UPDATE INSERTION AID INFORMATION

INSERTION AID INFORMATION

ACQUIRE INFORMATION — S101

EP 1 917 901 B1

# FIG.30

UPDATE TIMING OF
INSERTION AID INFORMATION
BY ANALYSIS PROCESSING
BLOCK 242

Tn-To>Tt

Tn-To<Tt

Tn-To<Tt

Tn-To<Tt

INFORMATION ACQUISITION
TIMING BY ANALYSIS RESULT
DISPLAY CONTROL BLOCK 243

DISPLAY CONTENTS OF
INSERTION AID INFORMATION

| DISTAL END STOP | DISTAL END STOP | DISTAL END STOP | |

EP 1 917 901 B1

| | | | |
|---|---|---|---|
| DISTAL END STOP | DISTAL END STOP | DISTAL END STOP | |
| DISTAL END STOP | DISTAL END STOP | DISTAL END STOP | |
| DISTAL END STOP | DISTAL END STOP | DISTAL END STOP | |

EP 1 917 901 B1

# FIG.32

*233*

| PROCESSING PROGRAM (CPU 230) | MEMORY |
|---|---|

*241*

**FRAME DATA ACQUISITION BLOCK**

*233a*

**FRAME DATA**

*251a* *251*

**ANALYSIS PROCESSING BLOCK**

*233d*

**PROCESSING SCRIPT**

**SCRIPT INTERPRETATION BLOCK**

*233b*

**ANALYSIS DATA**

**DISPLAY CHARACTERISTICS CHANGE PROCESSING BLOCK**

*233c'*

*251b*

**INSERTION AID INFORMATION**

**ANALYSIS RESULT DISPLAY CONTROL BLOCK**

*243*

# FIG.33

FLOW OF DATA IN FRAME DATA ACQUISITION BLOCK 241 AND ANALYSIS PROCESSING BLOCK 251

FLOW OF PROCESSING IN ANALYSIS RESULT DISPLAY CONTROL BLOCK 243

| FRAME DATA | PROCESSING SCRIPT | ANALYSIS DATA | INSERTION AID INFORMATION |
|---|---|---|---|

*233a*  *233d*  *233b*  *233c'*

GENERATE ANALYSIS DATA FROM FRAME DATA

ACQUIRE PROCESSING SCRIPT

CONDITION DETERMINATION  — Y — UPDATE INSERTION AID INFORMATION

N

GENERATE ANALYSIS DATA FROM FRAME DATA

ACQUIRE PROCESSING SCRIPT

CONDITION DETERMINATION  — Y — UPDATE INSERTION AID INFORMATION

N

GENERATE ANALYSIS DATA FROM FRAME DATA

ACQUIRE PROCESSING SCRIPT

INSERTION AID INFORMATION → ACQUIRE INFORMATION  *S101*

*S102*  Tn-To<Tt? — Y → DISPLAY INSERTION AID INFORMATION  *S103*

N

DELETE INSERTION AID INFORMATION  *S104*

INSERTION AID INFORMATION → ACQUIRE INFORMATION  *S101*

*S102*  Tn-To<Tt? — Y → DISPLAY INSERTION AID INFORMATION  *S103*

N

DELETE INSERTION AID INFORMATION  *S104*

INSERTION AID INFORMATION → ACQUIRE INFORMATION  *S101*

EP 1 917 901 B1

# FIG.34

```
/ * CONDITION DETERMINATION OF ANALYSIS DATA AND UPDATING OF INSERTION AID INFORMATION * /

var   mov_end.  mov_ratio;

mov_end=UPDPlayer. getScopeMoveEnd(); // ACQUIRE CURRENT PROPULSION AMOUNT OF HAND SIDE COIL
{
mov_ratio=UPDPlayer. getScopeMoveRatio(); // ACQUIRE CURRENT PROPULSION RATIO OF HAND SIDE COIL TO DISTAL END SIDE COIL
(EQUIVALENT TO Mo/Mn)

if(mov_end>=1.0{ // DETERMINE PUSHING OPERATION OF INSERTION PORTION WHEN PROPULSION AMOUNT OF HAND SIDE COIL IS NOT LESS THAN 1mm
    if(mov_ratio<0.1)&&(mov_ratio>-0.5)){
       UPDPlayer. setInfoText("DISTAL END STOP"); // UPDATE INSERTION AID INFORMATION
   } else if(mov_ratio<=-0.5){
       UPDPlayer. setInfoText("DISTAL END REVERSELY ADVANCE"); // UPDATE INSERTION AID INFORMATION
   }
} else if(mov_end<=-5.0){ // DETERMINE EXTRACTION OPERATION OF INSERTION PORTION WHEN PROPULSION AMOUNT OF HAND SIDE COIL IS NOT MORE THAN -5.0mm
    UPDPlayer. setInfoText(""); // RESET INSERTION AID INFORMATION
}
```

**EP 1 917 901 B1**

REFERENCES CITED IN THE DESCRIPTION

**Patent documents cited in the description**

- JP 2004147778 A
- JP 2004358095 A
- EP 1504712 A1